# EUROPEAN PATENT APPLICATION

(11) **EP 4 717 355 A1**
(43) Date of publication of application: **01.04.2026**
(21) Application number: 25172627.9
(22) Date of filing: 25.04.2025
(51) Int. Cl.: B01L 3/00, G01N 33/543

(54) **TEST DEVICE FOR TESTING MULTIPLE ANALYTES IN A LIQUID SAMPLE**

(30) Priority: 26.09.2024 CN 202411350959; 30.09.2024 US 202463700928 P
(71) Applicant: Hangzhou Biotest Biotech Co., Ltd., Yuhang District Hangzhou (CN)
(72) Inventor: Wu, Shujiang, Hangzhou (CN); Wu, Yiqi, Hangzhou (CN); Liu, Jiang, Hangzhou (CN); Hong, Liang, Hangzhou (CN); Hong, Luna, Hangzhou (CN); Ye, Chunsheng, Hangzhou (CN)
(74) Representative: Loo, Chi Ching

(57) **Abstract**

Disclosed in the present disclosure is a test strip for testing an analyte in a liquid sample and a device for testing an analyte in a liquid sample, including: a first lateral flow test element, including a first liquid transfer element (205), a first labeling element (202), and a first testing element (201); a second lateral flow test element, including a second liquid transfer element (102), a second labeling element (101), and a second testing element (103); and a sample receiving pad for receiving the liquid sample. The sample receiving pad is in fluid communication with the first liquid transfer element (205) and the second liquid transfer element (102). By using the device, multiple analytes in the same sample can be tested once, without mutual interference, thereby more simplifying and facilitating operations.

## Description

### CROSS-REFERENCE TO RELATED APPLICATIONS

The present disclosure claims priority to Prior Chinese Patent Application with application No. 2024113509598 and application date: 26 September 2024 and an US provisional application with application No. 63/700,928 with the application Date: 30 September 2024, the entire contents of which, including but not limited to the abstract, claims, specification, and drawings, are incorporated herein by reference in their entirety as part of the present application.

### BACKGROUND OF THE INVENTION

### Field of the Invention

The present disclosure relates to the technical field of immunochromatography, and in particular, to a lateral flow test strip including a labeling pad or a test device, and a test strip that can simultaneously test multiple analytes by using one sample or a device including the test strip.

### Description of the Related Art

The following background is used to help readers understand the present disclosure and should not be considered as prior art.

It becomes a very common method to collect a liquid sample, such as urine, by using a test device, and judge the presence of a specific analyte (such as drugs and/or metabolites thereof, or labels associated with diseases). Such test device generally requires that the sample should be collected in a sample container, and a relevant technician should insert a test strip, submerge part of the test strip into the sample, then take out the test strip and read the result. The technician may come into contact with the sample, which may endanger his/her health or contaminate the sample. To avoid such risk, it is necessary to add a closing lid to the sample collection container before the operation.

At present, there are a plurality of closed devices, for example, the devices disclosed in US patents US 4,976,923, US 5,429,804, and US 6,726,879. The above devices fix the test strip to the lid of the test device. In use, the container is turned over or tilted to make the sample infiltrate the test strip and get ready for the test. The US Patent Application US2003/0027359A1, published on February 6, 2003, discloses a urine test cup. After the opening of the urine test cup is covered by the cover, a post piston still needs to be pushed to move by a pushing rod, such that the liquid sample flows out of a cup chamber and wets the testing element. Patent Application 200510113977.5 published in China discloses a urine test cup. After the opening of the urine test cup is covered by the cover, the liquid flows from a collecting chamber to a testing chamber, and then the test is started. Another Patent Application 200480033286.8 published in China also discloses a urine test cup. After the opening of the urine test cup is covered by the cover, the test is started.

As many other sample collecting and testing devices are inefficient in extracting samples from the collection devices, they always have many problems, such as environmental pollution due to sample leakage, or testing results affected due to too few or too many samples collected, or complicated detection due to many operation steps. Many of these devices are also very complicated in their design and manufacture, for which quite expensive materials need to be used. Therefore, better methods and devices are needed to collect and detect samples.

Recently, such testing devices have been increasingly used by ordinary families or nonprofessional institutions. As these testing devices are specially designed for non-professionals, they need to simplify operation processes and ensure the accuracy of testing results. Therefore, testing devices with simple operation and accurate and reliable testing results are a necessity of the society at present.

At present, test strips or test cards and other testing materials developed with colloidal gold immunochromatography are widely used in medicines or ordinary people's homes. With these simple and fast test strips or test cards, for example, colloidal gold test strips for early pregnancy detection, ordinary people can obtain the testing results within one minute or ten-odd minutes at most. Therefore, it is of great significance to further improve these test strips or test cards. A lateral test strip is in contact with the liquid sample alone or by setting on a carrier, which absorbs liquid under capillary actions and allows the liquid to flow over the test strip to complete the test. Such test strip often includes an absorbent pad for contact with the liquid, a label pad to receive the liquid from the absorbent pad and dissolve the labeling substance on the absorbent pad, and a testing pad downstream to which the label flows to complete the test.

When these lateral flow test strips are used for testing, it is always hoped that samples are added once for testing multiple test indicators. This is more important in home testing. Adding the samples once is simple and convenient to operate, and the conditions or infection risks of many diseases can be evaluated by testing the multiple test indicators. In conventional test devices, such as the urine cup, approximately 10-50 milliliters of urine is collected, multiple test strips are arranged in parallel, and one or more analytes are tested correspondingly on each test strip, such that the multiple analytes can be tested. However, in this way, only two analytes are tested at most on each test strip, and exceeding this number may affect the test performance. Generally, the simplest method is to test one analyte on one test strip, for example, the US patent application with Publication No. US2022/0099668A1, where one sample can test multiple analytes by using a complex structural design. However, the multiple analytes are arranged on separate test strips, for example, this application includes five test strips that can simultaneously test five analytes. Although such a design can simultaneously test multiple analytes, the manufacturing costs are high, and substantial liquid samples are required. Particularly for home testing, it is always hoped that the operation is simple and multiple analytes are tested simultaneously.

Therefore, it is necessary to improve the existing test strip, so as to meet different test needs, make the testing results more accurate, and make the test strip especially suitable for home testing, with simpler operation and no influence on the testing results, while also requiring only a small amount of liquid samples.

### BRIEF SUMMARY OF THE INVENTION

The purpose of the present disclosure is to provide a test device. The test device can implement testing of multiple test indicators such as multiple analytes by using the same liquid sample, and these test indicators are all substantially completed on the same test strip, such that a plurality of different analytes can be tested on the same test strip. The test strip has two nonintersecting fluid flow paths, where each of the fluid flow paths has different reactions and different analytes, such that a plurality of different analytes are tested by using another new method.

Currently, the implementation of the combined test of multiple related analytes is a developing trend, and the home combined test of multiple test indicators is also a trend. Therefore, it is necessary to test multiple analytes once with a smaller amount of liquid samples when a home tester performs operations, such that the test results can be obtained more comprehensively, such as a combined test for respiratory diseases, e.g., Influenza A, Influenza B, novel coronavirus, respiratory syncytial virus (RSV), and other tests.

Therefore, in a first aspect of the present disclosure, provided is a device for testing an analyte in a liquid sample. The device includes a first lateral flow test element, including a first labeling element and a first testing element; and a second lateral flow test element, including a second labeling element and a second testing element.

In some embodiments, the device further includes a liquid flow splitting element. The liquid flow splitting element is simultaneously in fluid communication with the first testing element and the second testing element separately. In some embodiments, the liquid flow splitting element is arranged for receiving the liquid sample.

In some embodiments, the device further includes a first liquid transfer element. The first liquid transfer element is located upstream of the first labeling element. In some embodiments, the device further includes a second liquid transfer element. The second liquid transfer element is located upstream of the second labeling element. The liquid flow splitting element is respectively simultaneously in fluid communication with the first liquid transfer element and the second liquid transfer element. In this way, after the liquid flow splitting element obtains the liquid sample, a liquid is split into two flow paths to flow to the first testing element and the second testing element independently.

In some embodiments, the two flow paths respectively flow on the first testing element and the second testing element independently, and respectively flow through labeling pads and testing pads, and reactions are conducted independently on the testing pads on the two flow paths, such that different analytes can be tested respectively.

In some embodiments, a testing result control area is arranged on any one of the first testing element and the second testing element, and it is not necessary to arrange a testing structure control area on each of the two testing elements, thereby reducing one testing result control area and saving costs. Therefore, only a testing area is arranged on the first testing element, and a testing area and a testing result control area are arranged on the second testing element; alternatively, a testing area and a testing result control area are arranged on the first testing element, and only a testing area is arranged on the second testing element without a testing control area. The testing result control area is used to test whether a test strip is valid. Generally, a normal reaction occurs in the control area, indicating that a testing result in the testing area is valid; otherwise, indicating that a testing result in the testing area is invalid and cannot be regarded as a final testing result.

In some embodiments, the first liquid transfer element and the first labeling element are located on a same texture or material; alternatively, the second liquid transfer element and the second labeling element are located on a same texture or material. In some embodiments, the first liquid transfer element and the first labeling element are located on different textures or materials; alternatively, the second liquid transfer element and the second labeling element are located on different textures or materials. The textures or materials here all belong to porous absorbent materials.

If there is no liquid flow splitting element, instead, in some embodiments, the first liquid transfer element and the second liquid transfer element cover each other, and a covered and overlapped area between the first liquid transfer element and the second liquid transfer element is used to receive a liquid sample (the liquid flow splitting element can be omitted here); the liquid sample is applied to the overlapped area, the liquid is simultaneously in communication with the first liquid transfer element and the second liquid transfer element and respectively flow on the two liquid flow paths, thereby achieving respective and independent testing or assay. In some embodiments, the covered and overlapped area functions as a sample receiving pad. In some embodiments, one end of the first liquid transfer element and one end of the second liquid transfer element are located in a sample application hole. When the liquid sample is applied through the sample application hole, the first liquid transfer element and the second liquid transfer element are simultaneously in contact with the liquid sample, and the liquids are allowed to flow on their respective testing elements, to test or assay the analytes on their respective testing elements. In some embodiments, target analytes for two testing elements are different.

To enable the two flow paths to flow independently without interfering with each other, the traditional approach is to arrange two tests in parallel, with two testing elements spaced apart by a distance. However, it seems that an operator operates on one test strip. Actually, the respective independent flow paths are kept without interfering with each other. The present disclosure provides another approach. A summary description follows below.

In some embodiments, to be one testing element in appearance, the first testing element is located above the second testing element. The "above" here may mean that there is a certain distance between the first testing element and the second testing element, but the first liquid transfer element and the second liquid transfer element have an overlapped area to receive the liquid sample; alternatively, when the liquid flow splitting element serves as a liquid sample receiving function, the liquid flow splitting element is allowed to be simultaneously in fluid communication with the first liquid transfer element and the second liquid transfer element. In some embodiments, when there is spatial distance between the first testing element and the second testing element, the distance may range between 1-10 millimeters, such that a liquid flowing on the first testing element cannot flow onto the second testing element, thereby preventing mutual interference between the two liquid flow paths and respectively completing tests relatively independently. In some embodiments, such a physical distance may be set within a housing that is provided with a hole for applying the liquid sample. In the area of the hole for applying the liquid, the first liquid transfer element and the second liquid transfer element are allowed to have an overlapped area or the liquid distribution element is located below the hole, the first testing element is located in a first area of the housing and the second testing element is located in a second area of the housing, such that there is a physical distance between the first area and the second area. To look like one test strip, the first area is located above the second area, and there is a physical distance between the first area and the second area. In some embodiments, the first area is located upstream of the second area, or a projection of the first area is located upstream of the testing area of the second testing element. In this way, the first testing area of the first testing element is exposed, and the second testing area of the second testing element is also exposed. Viewed from the outside, the multiple analytes, e.g., 3-10 analytes, are tested on one testing element.

In other embodiments, the first labeling element is not in liquid communication with the second labeling element, or/and the first testing element is not in liquid communication with the second testing element. In some embodiments, the first labeling element is not in liquid communication with the second labeling element, and the first testing element is not in liquid communication with the second testing element. Alternatively, the first testing element is not in liquid communication with the second testing element.

In some embodiments, the first testing element is located above the second testing element. The "above" here may mean that the first testing element covers part of the surface of the second testing element. Therefore, in other embodiments, a partial area of the first testing element covers a partial area of the second testing element. In some embodiments, for testing performance and respective independent functions, only there is no mutual liquid communication between the first labeling element and the second labeling element, or/and there is no mutual liquid flow between the second testing element and the first testing element. Therefore, in some embodiments, the first labeling element covers the second labeling element, the first labeling element in fluid communication with the first testing element, and the second labeling element in fluid communication with the second testing element. In some embodiments, a non-absorbent isolation layer is arranged between the first labeling element and the second labeling element, and a non-absorbent isolation layer is arranged between the first testing element and the second testing element, thereby avoiding the interflowing of liquids. In some embodiments, the first labeling element and the first testing element cover the second labeling element but do not cover the second testing element, such that the isolation layers are only arranged between the second labeling element and the first labeling element as well as the second testing element, thereby exposing the first testing element and the second testing element. In some embodiments, the first testing element and the second testing element include one or more testing areas, each corresponding to the testing of one analyte. In some embodiments, the first lateral flow test element entirely covers upstream of the second testing area of the second lateral flow test element, such that the first lateral flow test element and the second lateral flow test element share a liquid distribution element that is configured to receive the liquid sample; alternatively, when the liquid transfer elements are present, the overlapped area between the first liquid transfer element and the second liquid transfer element serves as an area for receiving the liquid sample. Actually, in the lateral flow test, the labeling elements and the testing elements are critical areas. As long as no liquid flow occurs between the other two elements, the testing results are largely unaffected. Of course, to better avoid potential interference, an isolation layer is a preferred solution. Therefore, when the first lateral flow test element covers upstream of the second testing element of the second lateral flow test element, an isolation layer is arranged between the first lateral flow test element and the second lateral flow test element. The length of the isolation layer is substantially equal to or slightly less than that of the first lateral flow test element, e.g., 2-10 millimeters shorter, with a shortened area for receiving the liquid sample.

No liquid flow is made between the testing elements in such a way that the first testing element does not cover the second testing pad, such that the liquid does not flow from the first testing element to the second testing pad. For example, only the first testing element covers the second labeling element. In this way, an absorbent pad is arranged on the first testing element and can sufficiently absorb the liquid flowing on the first testing element, such that the liquid cannot flow onto the second testing pad. In some embodiments, when liquid transfer elements are arranged upstream of labeling elements, the first testing element covers the second labeling element and the second liquid transfer element, and non-water-absorbent isolation layers are arranged therebetween.

In some embodiments, a non-absorbent layer is arranged between the first lateral flow test element and the second lateral flow test element. Alternatively, a non-absorbent isolation layer is arranged between the first labeling element and the second labeling element. Alternatively, an isolation layer is arranged between the first liquid transfer element and the second liquid transfer element. In this way, the liquid flowing on the first lateral flow test element cannot flow onto the second lateral flow test element, or the liquid on the first liquid transfer element cannot flow to the second liquid transfer element.

In some embodiments, the first lateral flow test element and the second lateral flow test element can adhere together via the non-absorbent isolation layer. In some embodiments, when the length of the first lateral flow test element is smaller than that of the second testing element, or the length of the first lateral flow test element is substantially equal to a combined length of the second liquid transfer element and the second labeling element, such that the length of the isolation layer is equal to that of the first lateral flow test element, or at least, the length of the isolation layer is equivalent to a combined length of the first liquid transfer element, the first labeling element, and the first testing element. In this way, the two lateral flow test elements adhere together via the isolation layer. In some embodiments, the isolation layer has two ends, one thereof covers the second liquid transfer element, and the other end thereof covers the second labeling element. In this way, through the isolation of the isolation layer, the liquid sample transferred by the first liquid transfer element flows independently on the first lateral flow test element, and the liquid transferred by the second liquid transfer element flows independently on the second lateral flow test element.

In some embodiments, the liquid flow splitting element is located upstream of the first liquid transfer element and the second liquid transfer element, the liquid flow splitting element is in contact with the first liquid transfer element, thereby achieving liquid communication; and the liquid flow splitting element is in contact with the second liquid transfer element, thereby achieving liquid communication.

In some embodiments, the first lateral flow test element is located above the second lateral flow test element. In some embodiments, through adhering of the isolation layer, the first lateral flow test element is located above the second lateral flow test element. In some embodiments, through adhering of the isolation layer, the first lateral flow test element is located above the second liquid transfer element and the second labeling pad, instead of the second testing pad.

In some embodiments, the second liquid transfer element and the second labeling element may be combined into one absorbent element on which an area for treating a labeling substance is arranged. For example, the labeling substance is treated downstream of the absorbent element, and an upstream area serves as a liquid transfer element for liquid transfer.

In some embodiments, the first lateral flow test element covers the second lateral flow test element, wherein the first lateral flow test element does not cover a second testing element of the second lateral flow test element. In some embodiments, the first lateral flow test element covers the second lateral flow test element, wherein the length of the first lateral flow test element is substantially equal to the sum of the lengths of the second liquid transfer element and the second labeling element on the second lateral flow test element.

In other embodiments, the liquid transfer elements and the labeling elements are shared by both the first and second testing elements, and flow splitting can also be implemented on the testing elements. Therefore, the description that the first lateral flow test element is located above the second lateral flow test element includes sharing the labeling elements, and the testing elements implementing tests independently, i.e., the liquid is split on the labeling elements, such that the liquids flow independently on the first and second testing elements, thereby achieving the testing of multiple analytes. In some embodiments, the device includes a labeling area, a first testing element, and a second testing element, wherein the first testing element and the second testing element are in fluid communication with the labeling area. The first testing element is located above the second testing element. The "above" here also includes two specific embodiments. One of the two specific embodiments is that there is a physical separation or distance between the first testing element and the second testing element. The other specific embodiment is that the first testing element covers the partial area of the second testing element, with a non-absorbent isolation layer therebetween for isolation. In some embodiments, the first testing element covers part of the second testing element, and a non-absorbent isolation layer is arranged between the first testing element and the second testing element, thereby avoiding liquid interflowing between two different testing elements and interference with testing results. In some embodiments, to expose the testing areas on both the first testing element and the second testing element for facilitating reading, the length of the first testing element may be less than that of the second testing element, and no testing area is arranged in the covered area of the second testing element covered by the first testing element, instead, a testing area is arranged in an area of the second testing element not covered by the first testing element, such that the testing areas on both the first testing element and the second testing element are exposed. When the liquid flows in from the labeling element, one liquid path flows to the first testing element, and the other liquid path flows to the second testing element, such that reactions are respectively conducted in independent testing areas, thereby displaying or indicating testing results. In such embodiments, the liquid on the liquid transfer element and the labeling element is split on the labeling element and respectively flows to the first testing element and the second testing element, thereby achieving independent tests. The isolation of the non-absorbent isolation layer between the second testing element and the first testing element prevents liquid interflowing. In some embodiments, a liquid transfer element is upstream of a labeling area to receive the liquid sample. Of course, a liquid flow splitting element may be arranged upstream of the liquid transfer element. At this point, since the liquid is split on the labeling element, only one liquid flow path flows through the liquid transfer element or the liquid flow splitting element. The liquid flow splitting element may also serve as a liquid sample receiving element and transfer the liquid to the labeling element downstream or the liquid transfer element downstream.

In some embodiments, absorbent elements are arranged downstream of the first testing element or the second testing element, respectively, and allow the liquids to independently flow on the first lateral flow test element and the second lateral flow test element mainly by means of capillary force. Thus, the testing is completed.

In some embodiments, the device further includes a housing. The housing includes windows for reading testing results. The windows include a first window and a second window. The first window corresponds to the testing areas of the first testing element, and the second window exposes the testing areas of the second testing element. If the first or second testing element further includes a testing result control area, the testing result control area is exposed via the first or second window. In some embodiments, the housing further includes a liquid application hole. The liquid application hole corresponds to a liquid sample application area shared by the first lateral flow test element and the second lateral flow test element. For example, the liquid flow splitting element or the overlapped area of the first liquid transfer element and the second liquid transfer element is the shared liquid sample area, the liquid added via the application hole is received by the liquid flow splitting element to achieve flow splitting, or is received by the overlapped area of the first liquid transfer element and the second liquid transfer element to be transferred respectively by the first liquid transfer element and the second liquid transfer element to achieve flow splitting. In some embodiments, when flow splitting is on the labeling element, the liquid transfer element may be arranged to be in fluid communication with the labeling element; the application hole is formed in the liquid transfer element; and when the liquid flows to the labeling element, the liquid is split on the labeling element so as to respectively flow onto the first testing element and the second testing element. It can be understood that where flow splitting is implemented, where a non-absorbent isolation layer is arranged, thereby preventing interference or interflowing between the two liquid flow paths, and ensuring their independent flow. Of course, it can be understood that when the physical distance is used to prevent interflowing, there is a physical vertical spacial distance between the two flow paths starting from the position of flow splitting, such that the two flow paths can also flow respectively without interflowing and interference.

In some embodiments, the "element" in the first lateral flow test element and the second lateral flow test element can be replaced with "strip", thereby becoming the first lateral flow test strip and the second lateral flow test strip. Additionally, the "element" in other terms can be replaced with "pad", e.g., first testing pad, second testing pad, first labeling pad, second labeling pad, first liquid transfer pad, second liquid transfer pad, and liquid flow splitting pad. All these elements or pads here are made of porous absorbent materials, such that the liquid flows on the elements or pads by means of capillary force.

Therefore, in other embodiments, the first lateral flow test element includes a first testing element, or the second lateral flow test element includes a second testing element. The first testing element includes a first testing pad that includes a plurality of testing areas. The second testing element includes a second testing pad that includes a plurality of testing areas. The first labeling element includes a first labeling pad. The second labeling element includes a second labeling pad. The first liquid transfer element and the second liquid transfer element include a first liquid transfer pad and a second liquid transfer pad, respectively. The liquid flow splitting element includes a liquid flow splitting pad.

Therefore, in another aspect of the present disclosure, the present disclosure provides a device for testing an analyte in a liquid sample, including: a first lateral flow test strip, including a first liquid transfer pad, a first labeling pad, and a first testing pad; and a second lateral flow test strip, including a second liquid transfer pad, a second labeling pad, and a second testing pad, wherein one end of the first liquid transfer pad and one end of the second liquid transfer pad are arranged to be in liquid communication. In this way, when a liquid is dropwise added onto the first liquid transfer pad or the second liquid transfer pad, the liquid can flow along the respective liquid flow paths.

In some embodiments, the first lateral flow test strip is located above the second lateral flow test strip. The "above" here may mean that part of the first lateral flow test strip is located above a partial area of the second lateral flow test strip. One principle is that the standard is not to cover the testing areas on the second testing pad of the second lateral flow test strip, such that the testing areas of both the first testing pad and the second testing pad are exposed, thereby facilitating the reading of testing results on the testing areas. Therefore, in some embodiments, the first labeling pad is not in liquid communication with the second labeling pad, or/and the first testing pad is not in liquid communication with the second testing pad. In some embodiments, the first labeling pad is not in liquid communication with the second labeling pad, and the first testing pad is not in liquid communication with the second testing pad. In some embodiments, a non-absorbent isolation layer or a physical spatial distance is arranged between the first lateral flow test element and the second lateral flow test element. In some embodiments, the first lateral flow test strip and the second lateral flow test strip can adhere together via the non-absorbent isolation layer. At this point, on the one hand, the non-absorbent isolation layer prevents the liquid from interflowing between the two test strips; on the other hand, the first test strip and the second test strip adhere together via the adhering function of the non-absorbent isolation layer. In some embodiments, the isolation layer has two ends, one thereof covers the second liquid transfer pad, and the other end thereof covers the second labeling pad. In some embodiments, the first liquid transfer pad covers the second liquid transfer pad, achieving the liquid communication between the two transfer pads. A non-absorbent isolation layer is arranged in a partial area between the first liquid transfer pad and the second liquid transfer pad. Once the liquid sample contacts any of the transfer pads, the liquids can interflow at the areas without the isolation layer; once the liquids flow to one end of the isolation layer, liquid splitting is implemented. In some embodiments, the first liquid transfer pad is in contact with the second liquid transfer pad, thereby achieving liquid communication. In some embodiments, the contact portion is used for receiving the liquid sample. In some embodiments, the first lateral flow test strip covers the second lateral flow test strip, wherein the first lateral flow test strip does not cover the testing pad of the second lateral flow test strip. In some embodiments, the first lateral flow test strip covers the second lateral flow test strip, wherein the length of the first lateral flow test strip is substantially equal to the sum of the lengths of the second liquid transfer pad and the labeling pad on the second lateral flow test strip.

The present disclosure provides a device for testing an analyte in a liquid sample, including: a labeling pad or a labeling element, a first testing pad or a first testing element, and a second testing element or a second testing pad, wherein one end of the first testing pad or the first testing element and the second testing pad or the second testing element is in fluid communication with the labeling pad, and the first testing pad or the first testing element is located above the second testing pad or the second testing element.

In some embodiments, one or more testing areas are included on the first testing element, and one or more testing areas are included on the second testing element. In some embodiments, when the first testing pad is located above the second testing pad, the multiple testing areas on the first testing pad and the multiple testing areas on the second testing pad are exposed and unblocked. In this way, it is convenient to read the testing results of multiple testing areas on the first testing pad and the second testing pad. In some embodiments, a projection of the first testing element is located on the other areas on the second test strip without the testing areas. The other areas may include the other areas on the labeling pad, or the liquid transfer element, or the testing element or the testing pad on the second test strip. Of course, it is also possible that the projection of the entire first test strip is located on the other areas of the second test strip without the testing areas. For example, the entire first test strip is located on the other areas of the second test strip without the testing areas, which may still manifest as a spatial distance or an overlapping mode.

In some embodiments, the first testing area on the first test strip covers the other areas of the second test strip without the testing areas, such that a non-absorbent isolation layer is arranged between the covered areas. In this way, the first testing area and the second testing area are connected into an integrated structure. Viewed from the outside, it seems like one test strip, but it can test multiple analytes.

In some embodiments, the non-absorbent isolation layer includes an adhesive layer, by which the two testing strips adhere together. In some embodiments, the first test strip includes a first labeling pad located upstream of the first testing pad; the second test strip also includes a second labeling pad located upstream of the second testing pad; and the isolation layer is located between the two labeling pads. In some embodiments, if a first liquid transfer pad is further included upstream of the first labeling pad and a second liquid transfer pad is also included upstream of the second labeling pad, the isolation layer is also located between the two liquid transfer pads. When the first liquid transfer pad and the second liquid transfer pad have an overlapped area, which results in liquid communication, an isolation layer is arranged downstream of the overlapped area. The isolation layer is located downstream of the overlapped area, such that when the liquid sample contacts the overlapped area (with a liquid receiving function) or any liquid transfer pad, liquid communication between the two liquid transfer pads is achieved through the overlapped area. However, due to the presence of the isolation layer, liquid flow splitting is achieved at the isolation layer. A first liquid flow path flows from the first liquid transfer pad to the labeling pad downstream, then to the testing areas on the first testing pad downstream for testing and assay. A second liquid flow path flows from the second liquid transfer pad to the second labeling pad downstream, and then to the testing areas on the second testing pad downstream for analyte testing.

In a third aspect of the present disclosure, provided is a device capable of simultaneously testing multiple analytes. The device includes a first lateral flow test element, including a first liquid transfer element, a first labeling element, and a first testing element; and a second lateral flow test element, including a second liquid transfer element, a second labeling element, and a second testing element, wherein the first liquid transfer element is in liquid communication with the second liquid transfer element, a first labeling element is not in liquid communication with a second labeling element, and a second testing element is not in liquid communication with a first testing element.

In some embodiments, the second testing element is located below the first testing element, and the second testing element and the first testing element are connected into an integrated structure. In some embodiments, the first liquid transfer element and the second liquid transfer element are arranged for simultaneously receiving the liquid sample. In some embodiments, the device further includes a sample pad for receiving the liquid sample, and the sample pad is in fluid communication with the first liquid transfer element and the second liquid transfer element. In some embodiments, a non-absorbent isolation layer is arranged between the first lateral flow test element and the second lateral flow test element. In some embodiments, a non-absorbent isolation layer is arranged between the first labeling pad and the second labeling pad. In some embodiments, a non-absorbent isolation layer is arranged between the first testing pad and the second testing pad. In some embodiments, a non-absorbent isolation layer is arranged between the first labeling pad and the second labeling pad, and a non-absorbent isolation layer is arranged between the first testing pad and the second testing pad.

The present disclosure provides a method for testing an analyte in a liquid sample. The method includes: providing a test device, wherein the device includes: a first lateral flow test element, including a first liquid transfer element, a first labeling element, and a first testing element; and a second lateral flow test element, including a second liquid transfer element, a second labeling element, and a second testing element; allowing the first liquid transfer element and the second liquid transfer element to simultaneously receive the liquid sample; allowing part of a liquid to flow along the first lateral flow test element; and allowing the other part of the liquid to flow along the second lateral flow test element, wherein the liquid on the first lateral flow test element does not come into exchange with the liquid flowing on the second lateral flow test element.

The present disclosure provides a method for testing an analyte in a liquid sample. The method includes: providing a test device, wherein the device includes: a first lateral flow test element, including a first liquid transfer element, a first labeling element, and a first testing element; and a second lateral flow test element, including a second liquid transfer element, a second labeling element, and a second testing element; and allowing the first liquid transfer element and the second liquid transfer element to simultaneously receive the liquid sample, wherein liquids flow independently on the first lateral flow test element and the second lateral flow test element.

The present disclosure provides a method for testing an analyte in a liquid sample. The method includes: providing a test device, wherein the device includes: a first lateral flow test element, including a first liquid transfer element, a first labeling element, and a first testing element; and a second lateral flow test element, including a second liquid transfer element, a second labeling element, and a second testing element; allowing the first liquid transfer element and the second liquid transfer element to simultaneously receive the liquid sample; and allowing a liquid flowing through the first labeling element and the first testing element to be not substantially the same as a liquid flowing through the second labeling element and the second testing element.

In some embodiments, the first lateral flow test element is allowed to be located above the second lateral flow test element. In some embodiments, an isolation layer is arranged between the first lateral flow test element and the second lateral flow test element. In some embodiments, the first lateral flow test element and the second lateral flow test element are connected or adhere together via the isolation layer. In some embodiments, a non-absorbent isolation layer is arranged between the first labeling pad and the second labeling pad. In some embodiments, a length of the first lateral flow test element is allowed to be smaller than a length of the second lateral flow test element. In some embodiments, a length of the first lateral flow test element is allowed to be equal to or smaller than a sum of lengths of the second liquid transfer element and the second labeling pad on the second lateral flow test element. In some embodiments, the isolation layer is arranged between the first lateral flow test element and the second liquid transfer element and the second labeling pad on the second lateral flow test element. In some embodiments, the first lateral flow test element and the second lateral flow test element adhere into an integrated structure via the isolation layer.

In another aspect of the present disclosure, provided is a device for testing an analyte in a sample. The device includes a first liquid flow path and a second liquid flow path. The first liquid flow path and the second liquid flow path independently include a testing area, respectively. The testing area is arranged to be capable of testing one or more analytes in the liquid sample, wherein the first liquid flow path is located above the second liquid flow path. Alternatively, the first liquid flow path covers the second liquid flow path, wherein the first liquid flow path and the second liquid flow path respectively flow independently without liquid interflowing. In some embodiments, the first liquid flow path and the second liquid flow path share a labeling area that is located upstream of the first liquid flow path and the second liquid flow path, and the liquid on the labeling area is split into the first liquid flow path and the second liquid flow path.

In some embodiments, the labeling area further includes a liquid transfer element, and the liquid transfer element is located upstream of the labeling area and used for receiving the liquid sample. In some embodiments, the first liquid flow path includes a testing element, and a testing area is located on the testing element. In some embodiments, the second liquid flow path includes a testing element, and a testing area is located on the testing element.

### BRIEF DESCRIPTION OF THE DRAWINGS

Fig. 1 is a schematic diagram showing a three-dimensional structure of test strips in a test device according to a specific embodiment of the present disclosure.
Fig. 2 is a top view showing a test strip in a test device according to a specific embodiment of the present disclosure.
Fig. 3A is a schematic diagram showing a three-dimensional structure of test strips in a device according to a specific embodiment of the present disclosure.
Fig. 3B is a top view showing a test strip in a device according to a specific embodiment of the present disclosure.
Fig. 4 is a schematic diagram showing a three-dimensional structure of test strips in a test device according to a specific embodiment of the present disclosure.
Fig. 5 is a schematic diagram showing a three-dimensional structure of a test device according to a specific embodiment of the present disclosure.
Fig. 6 is a schematic diagram showing a three-dimensional structure of a test device according to a specific embodiment of the present disclosure.
Fig. 7 is a schematic diagram showing a three-dimensional structure of a test device according to a specific embodiment of the present disclosure.
Fig. 8 is a schematic diagram showing a three-dimensional structure of a test device according to a specific embodiment of the present disclosure (with test strips located in a housing).
Fig. 9 is a schematic diagram showing a three-dimensional structure of test strips arranged on a test card or box according to a specific embodiment of the present disclosure.
Fig. 10 is a schematic diagram showing a three-dimensional structure of test strips arranged on a test card or box according to another specific embodiment of the present disclosure.
Fig. 11 is a top view showing a test device according to a specific embodiment of the present disclosure.
Fig. 12A is a schematic diagram showing a three-dimensional structure of a test device according to a specific embodiment of the present disclosure.
Fig. 12B is a schematic diagram showing a three-dimensional structure of test strips according to a specific embodiment of the present disclosure.
Fig. 12C is a schematic diagram showing a three-dimensional structure of test strips in a test device according to a specific embodiment of the present disclosure.
Fig. 12D is a schematic diagram showing a three-dimensional structure of a test device according to a specific embodiment of the disclosure.
Fig. 13 is a top view showing a test device according to a specific embodiment of the present disclosure (displaying a positive result).
Fig. 14 is a schematic diagram showing a three-dimensional structure of test strips according to a specific embodiment of the present disclosure.
Fig. 15 is a schematic diagram showing a three-dimensional structure of test strips according to a specific embodiment of the present disclosure.
Fig. 16 is a schematic diagram showing a three-dimensional structure of a test device according to a specific embodiment of the present disclosure.
Fig. 17 is a schematic diagram showing a three-dimensional structure of a test device according to a specific embodiment of the present disclosure.

### DETAILED DESCRIPTION OF THE INVENTION

The structures involved in the present disclosure or the technical terms used are further explained below. Unless otherwise specified, they shall be understood and explained according to the general terms commonly used in the art.

### Testing

Testing means to assay or detect the presence or absence of a substance or material, including but not limited to, a chemical substance, an organic compound, an inorganic compound, a metabolite, a drug, a drug metabolite, an organic tissue, a metabolite of an organic tissue, a nucleic acid, a protein or a polymer. In addition, testing means testing the quantity of a substance or material. Further, assay also means immunodetection, chemical detection, enzymatic detection, and the like. The meanings of testing, detection, assay, and inspection in the present disclosure are used interchangeably.

### Samples

Biological liquid (for example, case liquids or clinical samples) samples or liquid samples can be tested by the test device of the present disclosure. The two terms, i.e., liquid samples or fluid samples, are used interchangeably. The liquid samples may be derived from solid or semi-solid samples, including excreta, biological tissues, and food samples. The solid or semi-solid samples may be converted to liquid samples by any appropriate methods, such as mixing, mashing, macerating, incubating, dissolving, or digesting the solid samples by enzymolysis in suitable solutions, such as water, phosphate solutions, or other buffer solutions. "Biological samples" include animal, plant, and food derived samples, including, for example, human or animal derived urine, saliva, blood and components thereof, spinal fluid, vaginal secretions, sperm, feces, sweat, secretions, tissues, organs, tumors, cultures of tissues and organs, cell cultures, and media. Preferably, the biological sample is urine. Preferably, the biological sample is saliva. Food samples include food processing substances, final products, meat, cheese, wine, milk, and drinking water. Plant samples include samples derived from any plants, plant tissues, plant cell cultures, and media. "Environmental samples" include samples derived from the environment (for example, liquid samples from lakes or other bodies of water, sewage samples, soil samples, groundwater, seawater, and waste liquid samples). The environmental samples may further include sewage or other wastewater.

Analytes in any sample can be tested by the suitable detection element or testing element (first and second testing elements), or the lateral flow test strip or the lateral flow test element, and the device including the testing element or the lateral flow test strip of the present disclosure. Preferably, the present disclosure is used to detect analytes in blood, saliva, nasal mucus, throat secretions, respiratory secretions, or urine, e.g., antigens such as drugs and viral bacteria. Preferably, the test device can be used to detect small molecular substances such as viruses and bacteria in saliva, throat, or nasal fluids. Any samples of the above forms may be collected using a collector (not shown), regardless of being solid or liquid at the beginning, provided that these liquids or liquid samples can be absorbed by an absorption element on the collector. The absorption element is generally located on the collector, such as a cotton swab, and a flocking swab. Generally, the absorption element here is made of an absorbent material, dry at the beginning, and can absorb liquid samples or fluid samples through the capillary or other characteristics of the absorption element material, such that the fluid samples can be kept in the absorption element. The absorption material may be any material capable of absorbing liquid, such as sponge, filter paper, polyester fiber, gel, non-woven fabric, cotton, polyester membrane, yarn, and flocking. When flocking swabs are used, flocking swabs as described in the following patents can be used to collect the fluid samples as a part of the present disclosure: US 8,114,027, US 8,317,728, US 8,979,784, US 9,011,358, US 9,173,779, US 10,327,741, AU 2004226798, JP 4579902, and ZL 200610099310.9. In some embodiments, the dry absorption element is hard; for example, the wet sponge becomes soft, and then can be compressed, thus releasing liquid. Of course, a sparse sponge, such as a sponge swab, can also absorb a small amount of liquid samples, such as 5-100 microliters. For example, a sponge cotton swab described in US Provisional Application 63/300,811, filed on Jan. 19, 2022, can also be used in the present disclosure as a specific embodiment of the collector.

Of course, the absorption element may be made of an absorbent material or a non-absorbent material. However, the absorption element is provided with holes, screw threads, and caves on which the samples can be collected. Generally, the samples are solid or semi-solid samples, and filled between screw threads and in the holes or caves for collection. Of course, optionally, the absorption element may be composed of some non-absorbent fibers and hairs, and these materials are used to scrape a solid, semi-solid or liquid sample, such that these samples can be retained on the absorption element. The samples collected by these collectors, after being processed, can be applied to the first or second lateral flow test element of the present disclosure for testing or assay.

### Downstream and upstream

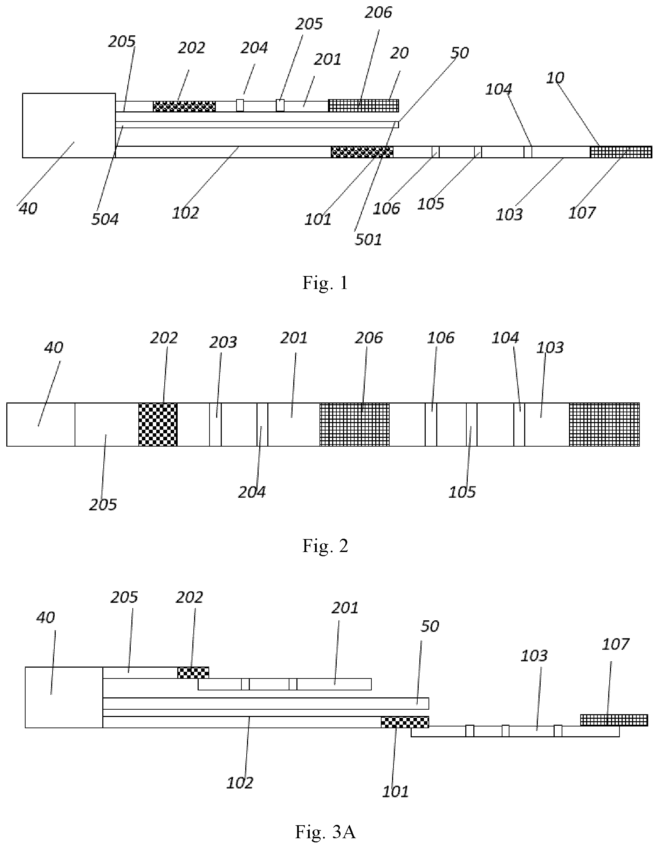

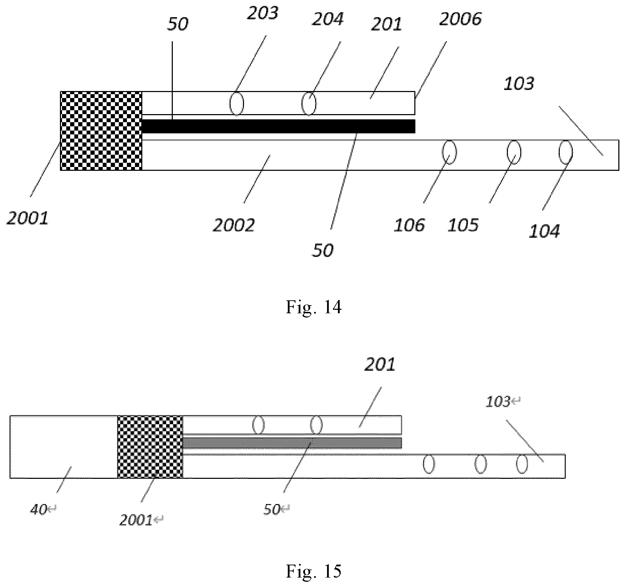

Downstream or upstream is divided according to a flow direction of a liquid. Generally, a liquid or fluid flows to a downstream area from an upstream area. The downstream area receives the liquid from the upstream area, and a liquid also may flow to a downstream area along an upstream area. Here, downstream or upstream is generally divided according to a flow direction of a liquid, for example, on some materials where capillary force is utilized to facilitate the flow of a liquid, a liquid may overcome gravity to flow towards an opposite direction to the gravity; and in this case, downstream or upstream is divided according to a flow direction of the liquid. For example, as shown in Fig. 1, the first lateral flow test element 20 mentioned in the present disclosure is provided with a first liquid transfer element 205, a first labeling element 202, a first testing element 201, and a first absorption element 206. The first liquid transfer element 205 is located upstream of the first labeling element 202. The first testing element 201 is located downstream of the first labeling element 202. The first absorption element 206 is located downstream of the first testing element 201. Generally, a fluid flows along a flowing direction of a testing element from upstream to downstream, for example, a fluid flows from the first liquid transfer element 205 to sequentially pass through the first labeling element 202, the first testing element 201, and the first absorption element 206. For another example, as shown in Fig. 15, a flow splitting element 40 is located upstream of a shared labeling element 2001, the shared labeling element 2001 is located upstream of both the first testing element 201 and the second testing element 103, and the first testing element adheres to the second testing element by a non-absorbent isolation layer 50. At this point, a liquid flows from the liquid transfer element 40 upstream into the shared labeling element 2001 downstream, is split at the shared labeling element 2001 to flow into the first testing element 201 and the second testing element 103 downstream, so as to be divided into two flow paths, i.e., a first liquid flow path located on the first testing element and a second liquid flow path located on the second testing element.

### Gas communication or liquid communication

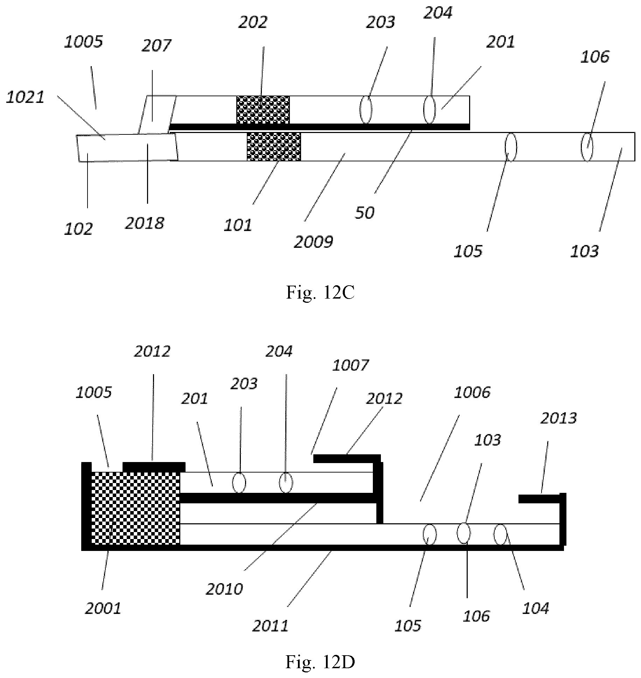

Gas communication or liquid communication means that a liquid or a gas can flow from one place to another. In the flow process, the liquid or the gas may pass through some physical structures that play a guiding role. The "passing through some physical structures" here generally means that the liquid passes through the surface of these physical structures or their inner space and flows to another place passively or actively, where passivity is usually caused by outer forces, such as flow under the capillary action and the action of air pressure. The flow here may also be a flow due to self-action (gravity or pressure) of the liquid or gas, and may also be a passive flow. The fluid under the action of air pressure may be a forward flow, or also a reverse flow; or a fluid is caused to flow to another position from a position under the action of air pressure. The communication here does not mean that a liquid or a gas is necessarily present, but indicates a connection relationship or state between two objects under some circumstances. If a liquid is present, it can flow from one object to another. Here, it means a state in which two objects are connected. On the contrary, if there is no liquid communication or gas communication between two objects, and if a liquid exists in or on one object but cannot flow into or onto another object, such a state is a non-communication, non-liquid communication or non-gas communication state. For example, as shown in Fig. 15, due to the presence of a non-absorbent isolation layer 50, a liquid on the first testing element 201 cannot flow to the second testing element 103, such that the first testing element is not in liquid communication with the second testing element. For another example, as shown in Fig. 12D, the first testing element 201 located on the first support plate 2010 is not in liquid communication with the second testing element 103 located on the second support plate 2011, this is, a physical spatial distance is set between the first support plate and the second support plate, such that the liquid cannot flow from the first testing element 201 to the second testing element 103. The shared labeling element 2001 is arranged upstream of the two testing elements. Due to the presence of the spatial distance, the first testing element is in liquid communication with the second testing element.

### Detachable combination

A detachable combination means that two components are connected in several different states or positional relationships. For example, with two components being physical components, they can be separated at the beginning and then connected or combined in an appropriate first case, and separated in an appropriate second case. Physically, such separation is spatial separation without contact. Alternatively, the two components are combined at the beginning, and can be physically spatially separated from each other when appropriate. Alternatively, two objects are separated at the beginning, combined to achieve a specified function if necessary, then separated, or later combined again for a purpose. In short, combination or separation of two components or two objects can be easily made and repeated many times. Of course, the combination or separation may also be single-use. In addition, such a combination may be a detachable combination between two components, or a two-by-two detachable combination between three or more components. For example, a first component, a second component, and a third component are provided, where a detachable combination is made between the first component and the second component or between the second component and the third component; and a detachable combination or separation is made between the first component and the third component. In addition, for the combination, two objects themselves can be detached or can be indirectly combined by other objects.

### Analyte

In examples where the analyte according to the present disclosure can be used, some semiantigen substances are involved and include drugs (such as drugs of abuse). "Drug of Abuse" (DOA) refers to the use of a drug (typically functions to paralyze the nerves) not directed to a medical purpose. Abuse of these drugs will lead to physical and mental damage, dependency, addiction and/or death. Examples of abuse of drugs include cocaine; amphetamine (AMP) (e.g., Black Beauty, white amphetamine tablets, dexamphetamine, dexamphetamine tablets, and Beans); methamphetamine (MET) (crank, meth, crystal, and speed); barbiturate (BAR) (such as Valium^{®}, Roche Pharmaceuticals, Nutley, and New Jersey); sedatives (i.e., a sleep aid medicine); lysergic acid diethylamine (LSD); inhibitors (downers, goofballs, barbs, blue devils, yellow jackets, and methaqualone); tricyclic antidepressants (TCAs, i.e. imipramine, amitriptyline, and doxepin); dimethylenedioxymethylaniline (MDMA); phencyclidine (PCP); tetrahydrocannabinol (THC, pot, dope, hash, weed, etc.); opiates (i.e., morphine (MOP) or opium, cocaine (COC), heroin, and hydroxydihydrocodeinone); and anxiolytic drugs and sedative-hypnotic drugs. The anxiolytic drugs are mainly used for relieving anxiety, tension, and fear, and stabilizing emotion, and have hypnotic and sedative effects. The anxiolytic drugs include benzodiazepines (BZO), atypical benzodiazepines (BZ), fused dinitrogen NB23C, benzazepines, ligands of BZ receptors, open-ring BZ, diphenylmethane derivatives, piperazine carboxylates, piperidine carboxylates, quinazolinones, thiazine and thiazole derivatives, other heterocycles, imidazole-type sedative/analgesic drugs (e.g., oxycodone (OXY) and methadone (MTD)), propylene glycol derivatives-carbamates, aliphatic compounds, anthracene derivatives, and the like. The test device of the present disclosure may also be used to test drugs belonging to a medical use but easy to be taken excessively, such as tricyclic antidepressants (imipramine or analogs) and acetaminophen. These drugs are metabolized into different micromolecular substances after being absorbed by human body. These micromolecular substances exist in blood, urine, saliva, sweat and other body fluids or in some body fluids.

For example, the analyte tested by the present disclosure further includes but is not limited to creatinine, bilirubin, nitrite, (nonspecific) proteins, hormones (for example, human chorionic gonadotropin, progesterone, follicle-stimulating hormone, etc.), blood, leucocytes, sugar, heavy metals or toxins, bacterial substances (such as proteins or carbohydrates against specific bacteria, for example, *Escherichia coli* 0157:H7, *Staphylococcus, Salmonella, Fusiformis, Camyplobacter genus, L. monocytogenes, Vibrio,* or *Bacillus cereus*) and substances related with physiological features in a urine sample, such as pH and specific gravity. The analyte may also be a bacteriovirus associated with respiratory infections and the like, such as influenza virus, coronavirus, RSV, adenovirus, human metapneumovirus (HMPV), coronaviruses such as SARS and MERS, rhinovirus, coronaviruses such as SARS-CoV, MERS-CoV, and SARS-CoV-2, coxsackievirus, echovirus, measles virus, herpes simplex virus (HSV), hantavirus, and Epstein-Barr (EB) virus. These viruses can be tested once by the test device or test strip of the present disclosure. Any other clinically interested analyte can be tested by lateral flow test in combination with the device of the present disclosure.

### Testing element

The "testing element" herein refers to an element that can be used to test whether a sample or a specimen contains an interested analyte. Such testing can be based on any technical principles, such as immunology, chemistry, electricity, optics, molecular science, nucleic acid science, and physics. The testing element may be part of a lateral flow test strip that can test multiple analytes. Of course, other suitable testing elements can also be used in the present disclosure. Generally, the testing element is provided with a testing area that generally contains or has an immobilized chemical substance such as an antibody. When a sample flows through the testing area, the antibody directly or indirectly captures analytes in the sample, such that the presence or absence or the quantity of the analytes is tested on the testing area.

Testing elements of various forms can be combined for use in the present disclosure (which will be explained in detail below). One form of the testing elements is a test strip or a lateral flow test strip. These test strips include a testing element. The testing elements used for analyzing the analyte (such as drugs or metabolites that show physical conditions) in the sample may be of various forms such as immunoassay or chemical analysis. The testing elements may adopt a non-competitive or competitive analysis mode. The test strip or lateral flow test strip generally includes a labeling element and a testing element. The labeling element includes a first receptor, such as a first antibody, that can specifically bind to the analyte. If the sample includes the analyte, the first receptor binds to the analyte to form a complex. The first antibody on the labeling element and the formed complex can flow along with the flowing of the liquid to the testing area of the testing element downstream. A second receptor, such as a second antibody, is immobilized on the testing area and can capture the complex, such that the quantity of the analytes can be obtained by testing the quantity of the complexes. Generally, the labeling element includes a labeling substance, such as a color substance (e.g., gold particles, latex particles, or dyes) or a luminescent substance (e.g., radioactive or fluorescent substances). The quantity or the presence or absence of the analytes can be represented by testing the quantity, color intensity, or luminescent intensity of color substances. Generally, the labeling element is located upstream of the testing element. When the labeling substance is shared, a first testing element and a second testing element may be included, as shown in Figs. 12D, 14, and 15.

In some embodiments, an absorbent material of a sample application area (e.g., a liquid sample transfer element), a reagent area (located on a labeling element), and a testing area (located on a testing element) of the lateral flow test strip are tested. Fluid or liquid samples are added to the sample application area and flow to the reagent area under the capillary action. If an analyte exists in the reagent area, samples will bind to the reagent. Then, the samples continue to flow to the testing area. Other reagents such as molecules that specifically bind to the analyte are immobilized in the testing area. These reagents react with the analyte (if any) in the sample and bind to the analyte in this area, or bind to a reagent in the reagent area. The label substance used to display a test signal is present in the reagent area or a separated labeling area.

In a typical non-competitive analysis mode, if a sample contains the analyte, a signal will be generated; and if not, no signal will be generated. In a competitive method, if no analyte exists in the sample, a signal will be generated; and if the analyte exists, no signal will be generated.

The testing element may be a test strip, which may be made of an absorbent material or non-absorbent material. The test strip may include a variety of materials for liquid sample delivery. One material of the test strip can cover another material thereof. For example, filter paper covers a nitrocellulose membrane. One area of the test paper may be of one or more materials, and the other area is made of one or more other different materials. The test strip can be stuck to a certain support or on a hard surface for improving the strength of holding the test strip.

The analyte is tested through a signal generating system. For example, by using one or more enzymes that specifically react with this analyte, and by using the above method of immobilizing a specific binding substance on the test strip, a combination of one or more signal generating systems is immobilized in the analyte testing area of the test strip. The substance that generates a signal may be in the sample application area, the reagent area, or the testing area, or on the whole test strip, and one or more materials of the test strip can be filled with this substance. A signal substance-containing solution is added onto the surface of the test strip or one or more materials of the test strip are immersed in the signal substance-containing solution. The test strip with the added signal substance-containing solution is dried.

The nitrocellulose membrane test strip is commonly used, that is, the testing element includes a nitrocellulose membrane (NC) as a testing element on which a specific binding molecule is immobilized (the specific binding molecule is immobilized in the testing area on the testing element) to display the testing result; and other test strips such as cellulose acetate membrane or nylon membrane test strips can also be used. For example, reagent strips or devices containing reagent strips are described in the following patents: US 4857453; US 5073484; US 5119831; US 5185127; US 5275785; US 5416000; US 5504013; US 5602040; US 5622871; US 5654162; US 5656503; US 5686315; US 5766961; US 5770460; US 5916815; US 5976895; US 6248598; US 6140136; US 6187269; US 6187598; US 6228660; US 6235241; US 6306642; US 6352862; US 6372515; US 6379620; and US 6403383. The test strips and similar devices with test strips disclosed in the above patents may be applied to the testing element or test device of the present disclosure for the test of an analyte, for example, the test of an analyte in a sample.

The testing reagent strips used in the present disclosure may be commonly referred to as lateral flow test strips, these testing reagent strips include testing elements, and the specific structures and test principles of these testing reagent strips are known to those skilled in the art in the prior art. A common conventional test strip includes a sample collection area or a sample application area, a labeling area, a testing area, and a water absorption area. The sample collection area includes a sample receiving pad. The labeling area includes a labeling pad. The water absorption area may include an absorbent pad. The testing area includes necessary chemical substances for testing the presence or absence of the analyte, such as immunoreagents or enzyme chemical reagents. The nitrocellulose membrane test strip is commonly used, that is, the testing area includes a nitrocellulose membrane, or the nitrocellulose membrane as a testing element includes a testing area where a specific binding molecule is immobilized on the nitrocellulose membrane to display the testing result; and other test strips such as cellulose acetate membrane or nylon membrane test strips can also be used. Of course, downstream of the testing area, there may also be a testing result control area; generally, test strips appear on the testing result control area and the testing area in the form of a horizontal line, namely, a test line or a control line. Such a testing reagent strip is conventional, and of course, other types of reagent strips for test by capillary action may also be used. In addition, generally, the testing reagent strip has dry chemical reagent components, such as immobilized antibodies or other reagents. When the reagent strip contacts a liquid, the liquid flows along the reagent strip with capillary action. With the flow, the dry reagent components are dissolved in the liquid, so as to go to the next area to treat the dry reagent in this area to react, so as to perform a necessary test. The liquid flow mainly relies on the capillary action. Here, all of the reagent strips can be applied to the test device of the present disclosure or can be disposed in contact with liquid samples in a testing chamber or used for testing the presence or absence of analytes in liquid samples that enter a testing chamber, or the quantity thereof.

In addition to the foregoing test strip or lateral flow test strip which is used for contacting the liquid samples to test whether the liquid samples contain analytes, the testing element of the present disclosure may be used as a test device by itself to test an analyte in a sample. Therefore, the test device here is equal to a testing element. For example, after mixed with a treatment solution, the fluid sample is tested with the testing element directly, specifically described as follows: when a receiving device is described to contact a fluid sample, the testing element may be used for testing alone. Of course, in some cases, the test device may be a test strip or any equipment or container including the test strip. The above description defines a single testing element. In the specific embodiments of the present disclosure, a combination of two or more testing elements is used for testing or detecting multiple analytes.

### Antibody

The antibody used in the present disclosure may be any antibody capable of specifically binding to the analyte in the sample. The antibody that can be used as a binding agent may be any antibody known to those skilled in the art. The "antibody" may be an immunoglobulin molecule and an antigen binding part of the immunoglobulin molecule, that is, a molecule including an antigen-binding site that specifically binds to an analyte, an analyte analog or a ligand ("immune reaction"). This term also includes derivatives of antibodies in which the binding ability is retained, and also includes any protein containing a binding domain that is homologous or largely homologous to the binding domain of an immunoglobulin. These proteins may originate from natural substances, or they may be partially or fully synthesized. An antibody may be monoclonal or polyclonal. An antibody may be a member of any immunoglobulin type, including any human immunoglobulin type: IgG, IgM, IgA, IgD, IgG, and IgE. An "antibody fragment" is a derivative of an antibody or a portion of an antibody that is less than the full length. The antibody fragment can retain at least one significant site of the binding ability of the full-length antibody. Examples of the antibody fragment include an antigen-binding fragment (Fab), an Fab', an F(ab')₂, a single-chain variable fragment (scFv), a variable fragment (Fv), a disulfide-stabilized Fv (dsFv) dimer, and an Fd fragment, but are not limited to the above. The antibody fragment may be generated by any means. For example, the antibody fragment can be generated by enzymatic or chemical cleavage of a complete antibody, or can also be generated by recombination of genes encoding partial antibody sequences. In other words, the antibody fragment can be generated by partial or complete recombination. The antibody fragment may be any single-chain antibody fragment. In other words, the antibody fragment may include a plurality of peptide chains linked to each other, for example, by disulfide bonds. The antibody fragment may also be any one of multi-molecular complexes. One functional antibody fragment typically includes at least about 50 amino acids, while more antibody fragments typically include at least about 200 amino acids. Single-chain Fvs (scFvs) are recombinant antibody fragments consisting only of a variable region of light chain (V_{L}) and a variable region of heavy chain (V_{H}) covalently bound to each other in a polypeptide chain. One of V_{L} and V_{H} has an amino terminal region. The length and composition of polypeptide chains are variable, and their length can bridge two variable domains to each other without significantly affecting the arrangement of atoms. Polypeptide chains are typically composed mainly of glycine and serine residue extensions, with some glutamic acid and lysine residues scattered to increase their solubility. The "dimer" refers to a bipolymer of singlestranded Fvs. The monomers of the dimer include peptide chains that are typically shorter than those of most single-chain Fvs, and they show a tendency to form the bipolymer.

The "Fv" fragment consists of one V_{H} domain and one V_{L} domain non-covalently linked to each other. The term "dsFv" here refers to an Fv including an intermolecular disulfide bond that stabilizes a V_{H}-V_{L} pair. The "F(ab')" fragment is a fragment of an antibody that is essentially identical to the fragment obtained by digesting an immunoglobulin (usually IgG) with pepsin at pH=4.0-4.5. This fragment can also be synthesized by recombination. The "Fab'" fragment is an antibody fragment that is essentially identical to the fragment obtained by reducing the disulfide bond linking the two heavy chains on the F(ab') fragment. The Fab' fragment can also be synthesized by recombination. The "Fab" fragment is an antibody fragment that is essentially identical to the fragment obtained by digesting an immunoglobulin (usually IgG) with papain. The Fab fragment can also be synthesized by recombination. The heavy chain fragment on the Fab fragment is the Fd fragment.

The antibody here can also be any kind of antibodies in the analyte, and these antibodies can also be used as the analyte.

### Test device

In some embodiments, the present disclosure provides a test device, as shown in Figs. 14-15, the test device includes a first testing element 201 and a second testing element 103. Each testing element includes a testing area, for example, the first testing element 201 includes two testing areas 203 and 204, and the second testing element 103 includes two testing areas 106 and 105. The testing area includes chemical reagents or immunoreagents that can display or test analytes in samples. These reagents can test the presence or absence of the analytes in the samples. For example, each testing area includes a second antibody that specifically binds to an analyte, and different testing areas have different second antibodies. In some embodiments, the first and second testing elements are made of an absorbent material, such as any filter paper and glass fiber, preferably a nitrocellulose membrane, a nylon membrane, and a cellulose acetate membrane. The chemical reagents or the immunoreagents are immobilized on the membrane.

In some embodiments, a non-absorbent isolation layer 50 is arranged between the two testing elements 201 and 103. One of the functions of the isolation layer is to prevent the liquid flowing on the first testing element 201 from flowing to the second testing element 103, such that independent test reactions are conducted on each testing element. It can also be considered that the first testing element 201 is provided with a first liquid flow path, and the second testing element 103 is provided with a second liquid flow path. The non-absorbent isolation layer 50 allows the two liquid flow paths to flow respectively independently without liquid exchange or flow. In some embodiments, the first testing element 201 covers the second testing element 103 via the isolation layer 50, but does not cover the testing areas 106 and 105 on the second testing element 103. In this way, when reaction, all the four testing areas (203, 204, 106, and 105) on the first testing element 201 and the second testing element 103 are exposed, facilitating the observation or machine reading of testing results of the testing areas. Consequently, in some embodiments, the isolation layer is arranged between the first testing element 201 and the second testing element 103 without covering or blocking the testing areas of the second testing element 103, that is, it covers the position of the second testing element that does not include the testing areas. Therefore, the second testing element 103 has a length greater than that of the first testing element, reserving a covered area 2002(can be looked as a projection are or a shadow area on the second test element), which is covered by the isolation layer and also indirectly covered by the first testing element 201 via the isolation layer. In this way, the first testing element 201 covers the covered area 2002 of the second testing element 103 that does not include the testing areas. In some embodiments, an isolation layer 2003 is a plastic film or has an adhesive layer, by which the first testing element 201 adheres to the second testing element 103 to serve as a whole. As the testing element is a thin layer, the second testing element is a thin layer, and the isolation layer is also a thin layer, the combined thickness of the three adhering layers is approximately 2-3 millimeters or 4-5 millimeters. Viewed from the top view, it seems that the complete test strip includes multiple testing areas. In this way, actually, tests are conducted independently respectively by independent testing elements, such that multiple analytes can be tested once, or it seems that one test strip has two independent liquid flow paths on which testing areas are arranged, and tests are conducted independently respectively in the testing areas on each liquid flow path.

In some embodiments, as shown in Figs. 14-15, the two testing elements are connected via a common labeling element 2001. In this case, as shown in Fig. 14, the labeling element serves as a shared labeling element 2001 for the first testing element 201 and the second testing element 103. A liquid is split on the shared labeling element into two liquid flow paths, i.e., a first liquid flow path flowing on the first testing element 201, and a second liquid flow path flowing on the second testing element 103. Due to the arrangement of the isolation layer, the two liquid flow paths flow independently respectively. If four different first antibodies are arranged on the labeling element, each first antibody can independently specifically bind to analytes in the liquid samples, such that independent complexes are formed. The complexes flow in two flow paths, for example, Influenza A and B, and novel coronavirus and RSV, thus forming an anti-Influenza A first antibody, an anti-Influenza B first antibody, an anti-novel coronavirus first antibody, and an anti-RSV first antibody. If a specific anti-Influenza A second antibody is immobilized on the testing area 203 of the first testing element 201 and an anti-Influenza B second antibody is immobilized on the testing area 204, an anti-novel coronavirus second antibody is immobilized on the testing area 105 of the second testing element, and an anti-RSV second antibody is immobilized on the testing area 106. If all the samples contain the analytes, the complexes can be captured on both the first testing area and the second testing area. Of course, if the first antibodies are conjugated with a labeling substance, such as a colored particle, colored particles will accumulate on all the testing areas of the first and second testing elements to represent the presence or absence of the analytes in the liquid samples.

Conversely, if multiple testing areas (more than 2) are continuously arranged on one testing element, it becomes difficult to solve the problem of mutual interference between test reagents. Since the tested analytes are different, the requirements for the reaction reagents processed on the testing elements are also different. If the multiple testing areas are arranged on the one testing element, the technical requirements for configuration are increased, and it becomes very difficult to avoid mutual influence. For example, if it is hoped that some testing areas are for immune reactions while other testing areas are for chemical reactions (e.g., redox reactions), and if the testing areas are all arranged on the same testing element, in any case, test reagents with two different reaction principles being arranged on the same testing element inevitably leads to interference between treatment processes, between reaction conditions, and between substances.

Even if all use the immune principle, for example, different antibodies are immobilized on the testing areas of one testing element, if the combined test of two or more analytes exists, e.g., 4-5 analytes, it has the defects or disadvantages as follows. First, 4-5 testing areas need to be treated on a single testing element, and the liquid needs to flow sequentially through the 4-5 testing areas on the single testing element. The liquid from the first testing area will flow to the subsequent testing areas, and the liquids from the at least 4 or 3 testing areas upstream will flow through the last testing area. These liquids necessarily affect the immune binding reaction in the last testing area (in some cases where the tested analytes are similar, they cannot be distinguished, for example, novel coronavirus has many subtypes, and influenza virus also has many subtypes, which may cause interference). Additionally, substances upstream may block capillary pores (testing elements are made of a porous absorbent material), which delays liquid flow. Second, the liquid samples need to pass through 4-5 areas that are separated, the entire testing area is relatively long, and sometimes the liquid samples are insufficient to flow from the first area to the last area, when there are few liquid samples or the a few volumes of the liquid sample. Furthermore, generally, testing results must be read within the specified time period to remain valid; typically, the testing results are read, e.g., within 3-5 minutes, from the start of the liquid being dropwise added to sample holes. If the time period is exceeded, the read testing results are invalid. If the liquid needs to sequentially flow through multiple testing areas, e.g., 4-5 or 6-7 testing areas that are provide on a single test element, it may take at least 1-3 minutes or more time(5-6 minutes )for the liquid to flow from the first testing area to the last testing area(5^{th} or 7^{th} test area). If the testing results are read in 3 minutes after the liquid is dropwise added, for example, it takes approximately 2 minutes for the liquid to flow through the labeling area upstream to flow to the first testing area, and after waiting for 1 minute, the testing result of the first testing area shall be read, however, at this time, the liquid may not yet flow to or may not pass through the last testing area( in fact, the liquid will take 5-6 minutes to pass through the last test are and read the rest result should be in 8-9 minutes for the 5^{th} or 7^{th} test area) ; obviously, it is too early to read the result of the last testing area at this time when read the first test area, which may confuse an operator which time should to read the test results on the last test area , or may miss the reading time for other test areas. Particularly for home self-testing, it is hoped that multiple items are tested with one sample, for example, when respiratory 6-plex (6 analytes), 5-plex (5 analytes), or 4-plex (4 analytes) assays, if these analytes are arranged on a single testing element, obviously, the interference between substances of the testing areas and the difficulty in production process need to be considered for production and manufacturing. Moreover, it is inconvenient for an operator, and particularly poses a huge challenge to the correct reading time of testing results of the testing areas.

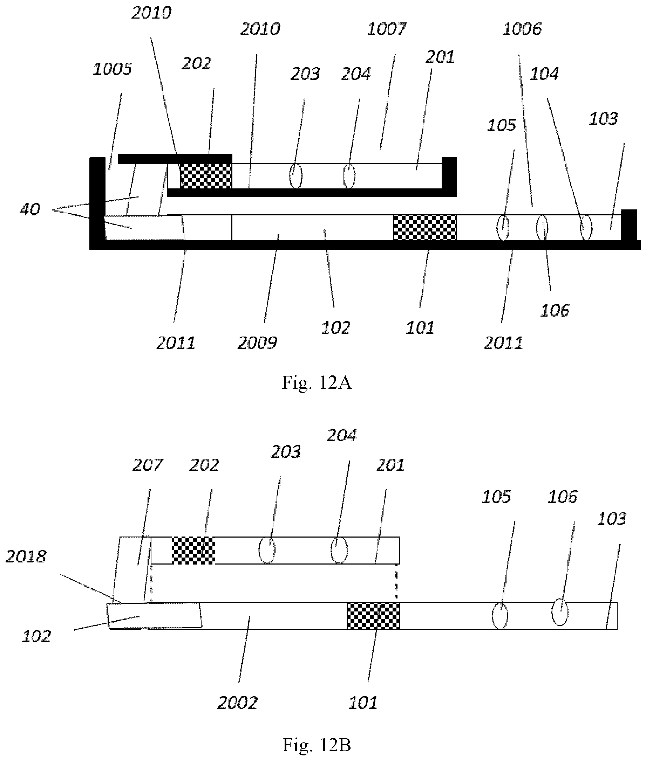

However, in the method of the present disclosure, two testing elements are overlapped (adhering via an isolation layer, as shown in Figs. 14-15) or combined from top to bottom (isolated by an isostructural base plate, as shown in Fig. 12A or 12D). When the upper testing element adheres to the lower testing element with a non-absorbent isolation layer arranged therebetween, the liquid flows on the two independent testing elements without interaction, thereby preventing interference. The reactions are conducted independently respectively, thereby reducing mutual interference. Additionally, controlling the reading time becomes easier. Although multiple analytes are tested, the liquids flow almost simultaneously separately on the two flow paths without mutual influence, such that the testing results are more accurate. Moreover, this avoids the interference with the reading of testing results for the operator, and the testing results can be read simultaneously. However, viewed from the outside, it seems that the tests are conducted on one test strip, making it more suitable for home testing. Therefore, in some embodiments, the testing reactions on the first testing element 201 and the second testing element 103 are both immune reactions, for example, receptors such as antibodies or antigens are immobilized on each testing area. In other embodiments, the testing areas 203 and 204 of the first testing element 201 are treated with antibodies, and the testing areas of the second testing element 103 are treated with chemical reagents for redox reactions. The two independent reactions do not interfere with each other, thereby increasing the possibility of different testing principles. Although the same liquid sample is used, the liquid flows independently on the first and second testing elements without mutual interference. In some embodiments, to facilitate the visual observation of results of the testing areas on the first and second testing elements (home testing), a labeling element 2001 is arranged upstream of the first and second testing elements, such that the first testing element and the second testing element share the same labeling element 2001. The labeling element 2001 is treated with labeled substances, such as antibodies or antigens, which are conjugated with colored labeling substances, such as gold particles, latex particles, and colored dyes. When the labeling element 2001 is present, one end 2004 of the isolation layer 2003 contacts the labeling element 2001, and the other end extends beneath a tail end 2006 of the first testing element 201. In this way, if the liquid flows onto the labeling element 2001, the liquid is split on the labeling element 2001 into two branches. One of the two branches flows onto the first testing element 201 and passes through the testing areas 203 and 204, and the other branch flows onto the second testing element 103 and passes through the testing areas 106 and 105, such that the same sample can be used for testing four different analytes, without exchange or flow of two liquid branches. The labeling element 2001 is shared by the first testing element 201 and the second testing element 103.

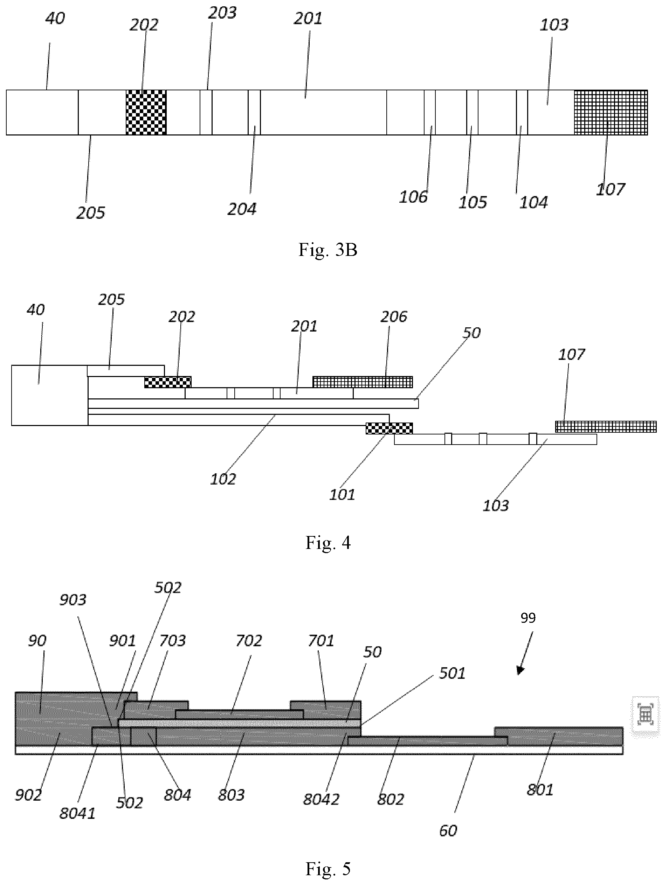

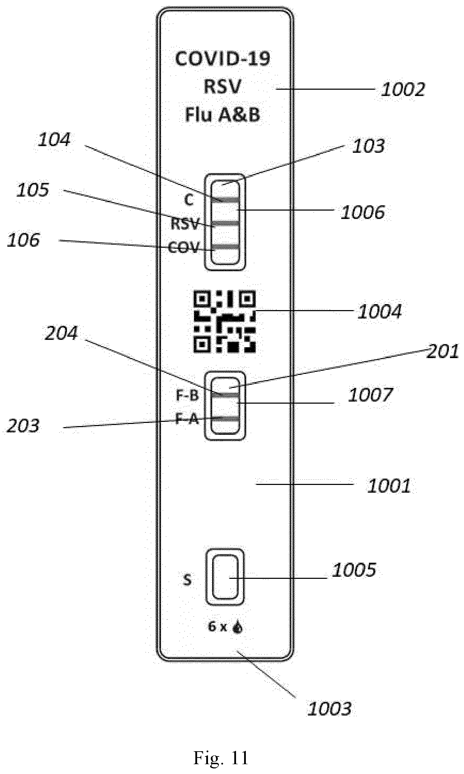

In some embodiments, a testing result control area is arranged on any one of the testing elements rather than on each of the two testing elements independently. Generally, in conventional techniques, for separate testing elements, generally, an independent testing result control area is arranged downstream of the testing areas to represent whether the testing elements are valid and whether the testing results of the testing areas are valid, for example, if no color appears in the testing result control area, the test is deemed invalid even if color appears in the testing result control area. In one embodiment of the present disclosure, the validity of testing results on two different testing elements can be controlled by arranging a testing result control area on only one of the testing elements. For example, as shown in Figs. 11 and 3B, a testing result control area 104 is arranged on the second testing element 103 to control or verify the validity of testing results on the testing elements. If no color appears in the testing result control area 104, the testing results of the testing areas 203 and 204 on the first testing element 201 and the testing results of the testing areas 106 and 105 on the second testing element are invalid. Conversely, the testing results on the two testing elements are valid.

In some embodiments, there is no liquid flow between the first testing element and the second testing element. In addition to adding a non-absorbent isolation layer, a physical distance may also be provided therebetween, for example, the first testing element 201 is located above the second testing element 103. The "above" here includes the above description where the first testing element 201 covers the surface of the second testing element 103, and may also include the case where the first testing element 201 is located above the second testing element 103 with a distance therebetween, such as 0.5, 1, 2, 3, 4, 5, 6, 7, 8, or 9 millimeters, preferably 2-3 millimeters. For example, as shown in Figs. 12A and 12D, as shown in Fig. 12A, a housing is provided. A first support plate 2010 and a second support plate 2011 located below the first support plate 2010 are arranged in the housing. There is a distance of approximately 2-3 millimeters or 1 millimeter between the second support plate and the first support plate. A first testing element 201 is arranged on the first support plate, a second testing element 103 is arranged on the second support plate, and there is a physical distance of 2-3 millimeters between the two testing elements. The projection of the first testing element is located in a projection area 2009 of the second testing element 103, and no testing area is arranged in the projection area 2009, instead, testing areas 105 and 106 are arranged downstream of the projection area, thereby exposing two testing areas 203 and 204 on the first testing element and two testing areas 106 and 105 on the second testing element. The exposure is by windows 1007 and 1006, which are on the same straight line. Viewed from the outside, it seems that four testing areas are arranged on one testing element. At this point, the non-absorbent isolation layer in the specific embodiments of the present disclosure does not need to be arranged between the first and second testing elements. Instead, the two support structures are arranged in the housing and separated from each other, such that there is no liquid flow between the first and second testing elements, and practically, two liquid flow paths are formed, i.e., a first liquid flow path located on the first testing element 201 and a second liquid flow path located on the second testing element. In this embodiment, a first labeling element 202 and a second labeling element 101 are arranged upstream of each testing element, and are treated with reagents with labeling substances corresponding to the testing areas. In some embodiments, a flow splitting element 40 is arranged upstream of the first labeling element 202, part of the flow splitting element is in communication with the first labeling element 202, and the other part is in communication with the second labeling element 101. In this way, when the flow splitting element 40 receives a liquid sample, for example, when the liquid sample is added via a sample application hole 1005, the liquid is split on the flow splitting element 40 into two liquid flow paths that flow independently respectively. At this point, one of the functions of the liquid flow splitting element is to serve as a liquid shared element, and the other function thereof is to receive a liquid sample. Of course, the flow splitting element is also made of a porous material, such as glass fiber, filter paper, and sponge.

For another example, as shown in Fig. 12D, in this specific embodiment, a labeling element 2001 is a shared element. The liquid flowing out from the labeling element 2001 flows to a first testing element 201 and a second testing element 103 respectively. The two testing elements are located on support plates 2010 and 2011, respectively. There is a physical distance between the two support plates. However, the first support plate is located above the second support plate, such that no liquid flow exists between the two testing elements, maintaining the independence property. For example, two testing areas 203 and 204 on the first testing element test Influenza A and B, respectively, and testing areas 105 and 106 on the second testing element 103 test novel coronavirus and RSV, respectively, so the shared labeling element 2001 is treated with an anti-Influenza A first antibody-conjugated gold particle, an anti-Influenza B first antibody-conjugated gold particle, an anti-RSV first antibody-conjugated gold particle, and an anti-novel coronavirus first antibody-conjugated gold particle, and an anti-Influenza A second antibody 203 and an anti-Influenza B second antibody 204 are immobilized on the testing areas on the first testing element; an anti-novel coronavirus second antibody and an anti-RSV second antibody are immobilized on the two testing areas 105 and 106 on the second testing element 103, respectively. The shared labeling element 2001 includes a chicken anti-mouse first antibody-conjugated gold particle, and a chicken anti-mouse second antibody is immobilized on a testing result control area 104 of the second testing element. In this structural configuration, a housing may be provided and divided into an upper layer and a lower layer. Only the first support plate 2010 is arranged on the first layer, and the second support plate 2011 is arranged on the second layer. The first layer is located 2-3 millimeters above the second layer, and the first support plate is located above the second support plate. Extending from the projection area of the first support plate 2010 projected onto the second support plate 2011, the second testing element 103 is arranged on the first support plate 2011, while the testing areas 105 and 106 or the testing result control area 104 is arranged on the extending area. A cover plate is provided. A first cover plate 2012 covers the first support plate 2010 and exposes (with a window 1007 arranged on the first cover plate 2012) the testing areas 203 and 204 of the first testing element 201. A second cover plate covers the extending area of the second support plate 2011 and exposes (with a window 1006 arranged on the second cover plate) the testing areas 105 and 106. As the spaced distance between the two sides is very small and they are located on the same straight line, viewed from the outside of the housing, it seems that one testing element displays multiple testing results; actually, two different testing elements display different testing results. As shown in Fig. 11, viewed from the top view, it seems that the display window displays multiple analytes on one testing element. Actually, a first window 1007 displays the testing results of two testing areas 203 and 204 on a first testing element 201, and a second window 1006 displays the results of testing areas 105 and 106 and a testing result control area 104 on a second testing element 103. Viewed from the outside, it seems that the second window 1006 is located downstream of the first window 1007, and they are also considered as different areas on one testing element. Actually, the test is completed by different liquid flow paths on the two different testing elements.

A liquid sample application hole 1005 corresponds to different elements under different conditions. For example, as shown in Figs. 12A-12D, 14, and 15, a liquid sample application hole 1005 may correspond to a shared labeling element 2001 (Figs. 14 and 12D), or a shared liquid flow splitting element 40 (Fig. 12A), or an intersection area 2018 of two shared liquid transfer elements as shown in Fig. 12B. In this embodiment, a first labeling element 202 and a second labeling element 101 are independently utilized by a first testing element 201 and a second testing element 103, respectively, without being shared. A first liquid transfer element 207 is arranged upstream of the first labeling element 202, a second liquid transfer element 102 is arranged upstream of the second labeling element 101, and the intersection area 2018 of the two liquid transfer elements corresponds to the sample application hole 1005. After the liquid sample is added, the liquid is split into two flow paths inside. One of the two flow paths is to flow along the first liquid transfer element 207 to the first labeling element 202 and then to the first testing element 201. The other flow path is to flow to the second liquid transfer element 102 and then flow through the second labeling element 101 and the second testing element 103. Of course, for example, in Fig. 12C, a first liquid transfer element 207 and a second liquid transfer element 102 share an intersection or overlapped area 2018. However, the second liquid transfer element 102 extends forward, and a sample application hole is formed in an extending area 1021. The liquid sample flows into the extending area 1021 and then flows to the overlapped area 2018. At this point, part of the liquid sample flows along the first liquid transfer element 207 onto a first labeling element 202 and flows onto a first testing element 201, and the remaining part flows along the second liquid transfer element 102 onto a second labeling element 101 downstream and flows onto a second testing element 103. At this point, the liquid transfer elements serve as shared liquid pads for flow splitting. In this way, the liquid is added once. Viewed from the outside, it seems that multiple analytes are tested on one testing element, for example, 3, 4, or 6-7 analytes are tested. Actually, multiple analytes are tested on two liquid flow paths, avoiding some defects of conventional test strips and providing another thought for achieving the once combined test of multiple analytes. Although the present disclosure belongs to the combined test of multiple analytes, the present disclosure provides a new approach and method to achieve similar and identical objectives, avoiding the defects of conventional tests, more saving costs, more facilitating operations, and achieving more friendliness and convenience. Additionally, the two testing elements independently conduct reactions, and the liquid flows independently, such that the reading can be conducted almost simultaneously.

As shown in Fig. 12C, in some embodiments, a first labeling element 202 is arranged upstream of a first testing element 201, a second labeling element 101 is arranged upstream of a second testing element 103, and the first testing element and the first labeling element are located on a partial area (i.e., on a projection area or a shadow area 2009 of the first labeling element 202 and the first testing element on the second labeling element and second testing element) of the second testing element. No testing area is arranged on the partial projection area 2009 on the second test element, such that the first testing element and the first labeling element cover the projection area 2009. The second labeling element 101 is located within the projection area 2009 of the first testing element 201 and the first labeling element 202 on the second testing element. Consequently, when the first testing element covers the second testing element, the second labeling element 101 is covered by the first testing element. To prevent the liquid on the first testing element 201 and the first labeling element 202 from flow or exchange with the second testing element 103 and the second labeling element 101 below, a non-absorbent isolation layer 50 is arranged therebetween, that is, the non-absorbent isolation layer covers the projection area 2009, and the length of the projection area 2009 is the sum of the lengths of the first labeling element 202 and the first testing element 201. In this way, the liquid transfer elements can be shared, but the labeling elements and the testing elements are not shared, such that the liquid after passing through the liquid transfer elements is split into two independent liquid flow paths.

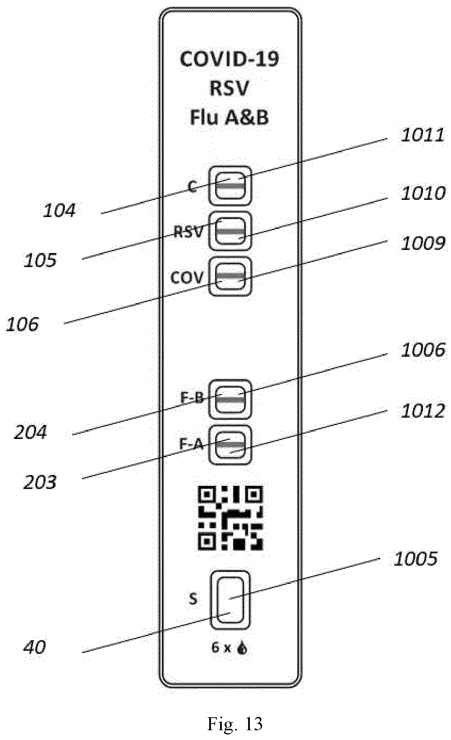

In some embodiments, for example, as shown in Fig. 1, a first lateral flow test strip 20 is arranged, and a first liquid transfer element 205, a first labeling element 202 downstream, and a first testing element 201 downstream are sequentially arranged on the first lateral flow test strip 20. Optionally, a first absorbent element 206 is arranged downstream of the first testing element. A second lateral flow test strip 10 is further included and includes a second liquid transfer element 102; a second labeling element 101 is arranged downstream of the second liquid transfer element 102; a second testing element 103 is arranged downstream of the second labeling element. Optionally, an absorbent element 107 is arranged downstream of the second testing element 103. The first liquid transfer element 205 and the second liquid transfer element 102 are in connection or fluid communication with a liquid flow splitting element 40. The first lateral flow test strip 20 is located upstream of the second lateral flow test strip, the length of the first lateral flow test strip is less than or equal to the length of a liquid flow path formed by the second liquid transfer element 102 and the second labeling element 101 of the second lateral flow test strip 10, or the length of the first lateral flow test strip 20 is less than that of the second lateral flow test strip 10. When the first lateral flow test strip covers the second lateral flow test strip, the first lateral flow test strip does not cover testing areas 106 and 105 and a testing result control area 104 of the second testing element. In this way, testing areas 203, 204, 106, and 105 of the two lateral flow test strips 20 and 10 are all exposed, facilitating the reading of testing results. Other areas may be hidden with a housing outside (as shown in Fig. 11 or Fig. 13). The liquid flow splitting element 40 serves as a shared element and serves as a liquid sample receiving element. It can be understood that the shared liquid flow splitting element 40 here is located below the sample application hole 1005 for receiving the liquid sample. In some embodiments, to prevent the liquid exchange or interflowing between the two lateral flow test strips, a non-absorbent isolation layer is arranged on partial areas of the two lateral flow test strips. The length of the non-absorbent isolation layer is basically or substantially equal to that of the first lateral flow test strip 20. In this way, due to the action of the isolation layer, the liquid flowing on the first lateral flow test strip 20 cannot flow onto the second lateral flow test strip 10, ensuring the independent flow of the liquid on each test strip and sharing the liquid on the liquid flow splitting element 40. In some embodiments, one end 504 of the isolation layer 50 is connected to the flow splitting element 40, and the extended length of the other end 501 is equal to that of the first lateral flow test strip 20. Viewed from the top view, such a combined test strip seems like a single test strip, as shown in Fig. 2, the liquid flow splitting element 40 serves as a liquid receiving area or a liquid receiving element. A first liquid transfer element 205 is downstream of the liquid flow splitting element. A first labeling element 202 is downstream of the first liquid transfer element. A first testing element 201 is downstream of the first labeling element. A first absorbent element 206 (may also be omitted) is downstream of the first testing element. A second testing element 103 is downstream of the first absorbent element. A second absorbent element 107 is downstream of the second testing element. The second liquid transfer element 102 and the second labeling element 101 of the second lateral flow test strip are covered and blocked by the first lateral flow test strip above to be not exposed or displayed. Such a test strip is placed in a housing, for example, as shown in Fig. 11, it can be seen that the sample application hole 1005 corresponds to the liquid flow splitting element 40, testing areas 204 and 205 on the first testing element 201 on the first lateral flow test strip 20 are exposed by a window 1007, and testing areas 106 and 105 and a testing result control area 104 on the second testing element 103 on the second lateral flow test strip 10 are exposed by a window 1006.

In some embodiments, for example, as shown in Fig. 3A, a first liquid transfer element 205 and a second labeling element 202 are located on the same material, which has only two areas, one with a labeling substance, and the other one without a labeling substance serving as a liquid transfer function. Generally, the same material is a porous absorbent material, one end of the material laps over the first testing element 201, and at this point, an absorbent pad 206 is omitted. Similarly, a second liquid transfer element 102 and a second labeling element 101 are located on the same material, which has only two areas, one with a labeling substance, and the other one without a labeling substance serving as a liquid transfer function. The two parts serving as the liquid transfer function share the liquid flow splitting element 40. It can be understood that any of the above-mentioned elements can be replaced with a "pad", all of which are made of porous absorbent materials. The porous absorbent materials have a capillary action, enabling liquids (e.g., liquid samples) to flow on these porous materials. A non-absorbent isolation layer is arranged between the two test strips, similar to the arrangement shown in Figs. 1-2, with the only difference being that a first absorbent pad is omitted on the first test strip. The top view of such a combined test strip is shown in Fig. 3B. Viewed from the outside, it seems that five areas, i.e., four testing areas and one testing result control area 104, are arranged on one test strip. Actually, the test strip is combined by partially overlapping two different test strips 10 and 50. Part of the liquid flowing into the liquid flow splitting element 40 flows along the first test strip 50, while the other part of the liquid flows independently along the second test strip 10.

In some embodiments, as shown in Fig. 4, a first liquid transfer element and a first labeling element are not made of the same material, the first labeling element 202 is made of an independent material, and the first liquid transfer element 205 is also made of an independent material. The first liquid transfer element 205 laps over the first labeling element 202, the first labeling element 202 laps over the first testing element 201, and the absorbent element 206 laps over the first testing element 201. The isolation layer 50 covers the second liquid transfer element 102 and the second labeling element 101 of the second lateral flow test strip, completely. The first lateral flow test strip covers the isolation layer 50. One end of the second liquid transfer element 102 and one end of the first liquid transfer element 205 share the liquid flow splitting element 40. Due to the presence of the isolation layer, which is non-absorbent or waterproof, the liquid cannot flow from the first lateral flow test strip onto the second lateral flow test strip, avoiding mutual interference.

In some embodiments, as shown in Fig. 5, a combined test strip 99 is provided. The test strip 99 includes a first labeling pad 703 with one end lapping over a first testing pad 702; and a first absorbent pad 701 lapping over one end of the first testing pad 702 to form a lateral flow test strip with a testing function. A second test strip has a relatively long second liquid transfer pad 803 on which a second labeling area 804 with a labeling substance is arranged. One end of the second liquid transfer pad 803 laps over a second testing pad 802, and a second absorbent pad 801 laps over the second testing pad 802. Part of one end 901 of the liquid shared pad 90 laps over the other end of the first labeling pad 703, and part of a lower part 902 laps over a partial area 8041 of the second liquid transfer pad 803. One end 501 of the isolation layer 50 aligns with the tail end of the first absorbent pad 701, and the other end 502 is located at a lower portion of one end 901 of the liquid shared pad 90 but is longer than the first labeling pad 703. However, the partial area 8041 of the second liquid transfer pad 803 is not covered by the isolation layer but is in contact with the liquid shared pad 90, whereas the second labeling area 804 is entirely covered by the isolation layer. In this way, after the liquid shared pad 90, serving as a sample application pad, receives the liquid sample, part of the liquid flows directly onto the first labeling pad 703. Since one end 502 of the isolation layer is longer than the first labeling pad 703, the liquid on the first labeling pad 703 cannot flow onto the end 8041 of the second liquid transfer pad 803. Instead, the liquid passes through the end 8041 of the second liquid transfer pad 803, flows into and through the second labeling area 804, and flows to the second testing pad 802 downstream. A testing area is arranged on the first testing pad 702, and a testing area is also arranged on the second testing pad 802. Both the testing areas on the two testing pads are exposed and uncovered, such that the testing results can be read from the top view.

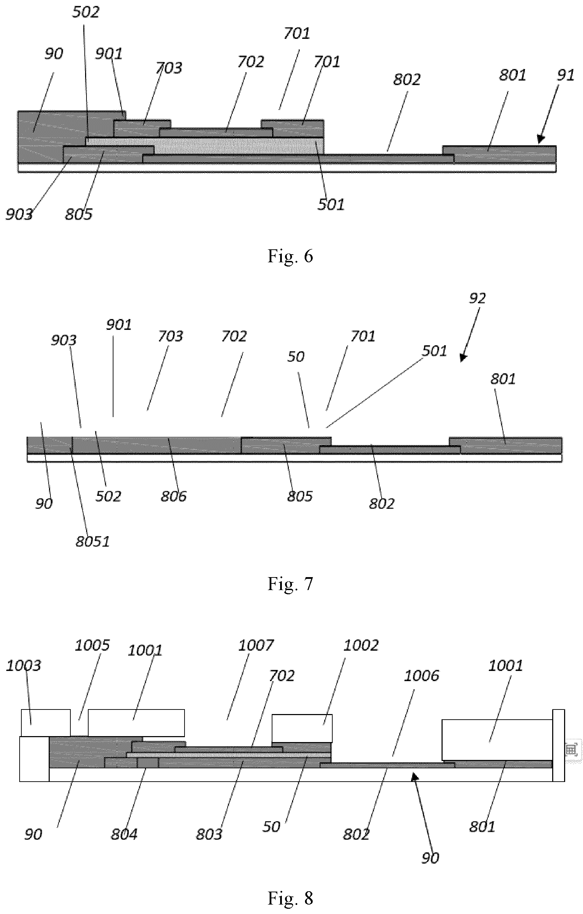

In some embodiments, for example, another test strip 91 shown in Fig. 6, unlike the example shown in Fig. 5, one end of a second labeling pad 805 laps over a second testing pad 802, and the other end is overlapped by a lower portion 902 of a liquid flow splitting element 90 to form a covered area 903, such that the shared or flow splitting element 90 is in fluid communication with the second test strip via the covered area 903. One end 502 of the isolation layer covers the second labeling pad 805 but does not over the covered area 903; however, a first labeling pad 703 covers the isolation layer, with an end shorter than an end 502 of the isolation layer (or the end 502 of the isolation layer is longer than the end of the first labeling pad 703), such that the first labeling pad 703 and the second labeling pad 805 are separated by the isolation layer 50 while both share the liquid flow splitting pad 90. The second testing pad 802 here is set longer. The isolation layer covers part of the surface of the second testing pad. However, no testing area is arranged on the covered part; instead, a testing area is arranged on the position that is not covered by the isolation layer and is exposed. Of course, an absorbent pad 801 may be arranged at the tail end of the second testing pad 802. Optionally, an absorbent pad 701 may be arranged at the tail end of the first testing pad 702. In this way, after the two different test strips are combined, the device seems like one test strip overall, but actually, the device is formed by combining two different test strips.

In some embodiments, for example, a test strip 92 shown in Fig. 7, unlike that shown in Fig. 6, the second test strip includes a liquid transfer pad 806 and a second labeling pad 805. One end of the second labeling pad 805 laps over a second testing pad 802. One end of the liquid transfer pad 806 laps over one end of the second labeling pad 805. An isolation layer 50 covers part of the surface of the liquid transfer pad 806, but liquid communication is formed at the position 903 where the exposed surface laps over a liquid flow splitting pad 90, such that the liquid can flow from the flow splitting pad 90 onto the liquid transfer pad 806. Moreover, the end of one end 502 of the isolation layer is longer than the end of a first labeling pad 703, such that the liquid on the flow splitting pad 90 is in liquid communication with the first labeling pad 703 but the liquid cannot flow onto the liquid transfer pad 806 below. Thus, the liquid on the flow splitting pad flows along two flow paths. The flow splitting pad 90 serves as a shared liquid pad. The isolation layer 50, being a waterproof film or a thin layer, covers the entire second labeling pad 805 and part of the liquid transfer pad 806. At this point, for the flow splitting pad 90, the flow splitting pad 90 functions as a liquid transfer pad and a liquid sample receiving pad. After the liquid is received by the flow splitting pad 90, part of the liquid flows through the first labeling pad 703 onto a first testing pad 702, and the other part of the liquid flows through the liquid transfer covered area 903 onto the liquid transfer pad 806, flows through the second labeling pad 805, and then flows through testing areas on the second testing pad 802. Since the length of the covered area of the isolation layer is equal to or greater than that of the first test strip, the liquid on the first test strip cannot flow onto the second test strip below. However, the testing areas on both the first testing pad 702 and the second testing pad 802 are exposed, such that the testing results on the testing areas can be read. Since these pads are formed by combining very thin sheets (with a thickness of 2-3 millimeters), the entire test strip seems like one test strip, provided inside with two liquid flow paths that flow independently for testing and assay. After all, each pad is approximately 1-2 millimeters thick, and the testing pads, if being nitrocellulose membranes, are approximately 1 millimeter or micron-level in thickness. Thus, the entire test strip is approximately 2-3 millimeters thick, and seems similar to the conventional test strips, viewed from the outside.

As shown in Figs. 1-10, 12C, 14, and 15, an isolation layer, on one hand, prevents liquid exchange between partial areas or partial elements of upper and lower test strips. On the other hand, an upper surface (in contact with the first test strip) and a lower surface (in contact with the second test strip) of the isolation layer can also be coated with adhesive layers, by which the separated upper and lower parts can adhere together to form an integrated structure. The isolation layer here may be made of a plastic film, a double-sided adhesive, a plastic thin sheet, a metal thin sheet, or an alloy thin sheet, or the isolation area is coated with a waterproof thin layer, e.g., a hydrophobic layer such as hydrophobic adhesive. Of course, in some embodiments, a porous absorbent material may be used as an isolation layer, but the porous absorbent material is treated with a hydrophobic layer, such that the liquid flows on the surface of these hydrophobic layers but cannot permeate, thus preventing liquid exchange between the upper and lower porous absorbent materials. The modes of arrangement of the isolation layer here are functionally categorized. The isolation layer may be arranged between a first testing element and a second testing element to prevent liquid exchange (Figs. 14 and 15), and at this point, a labeling element or labeling pad may be shared and the liquid is split on the labeling pad or labeling element. The isolation layers may be arranged between a first labeling pad and a second labeling pad as well as between a first testing pad and a second testing pad (Figs. 12C, 10, 9, 8, 7, 6, and 1-4) and prevent the liquid exchange between the labeling pads as well as between the testing pads, and at this point, a liquid transfer element or a flow splitting element arranged upstream of the labeling pads may be shared. The isolation layers may also simultaneously prevent the liquid exchange between the liquid transfer pads, between the labeling pads as well as between the testing pads, and at this point, the flow splitting element is shared by two flow paths. Of course, the isolation layers may also simultaneously prevent the liquid exchange between the liquid transfer pads, between the labeling pads, between the testing pads as well as between the absorbent pads, and at this point, only the liquid flow splitting element is shared. Thus, by using different modes, the areas and modes where the isolation layers prevent liquid exchange are different. Of course, it is more important to prevent liquid exchange between testing elements or testing pads, or/and labeling elements and labeling pads, for example, liquid exchange between testing areas of a first testing element and testing areas of a second testing element, or between a fist labeling element and a second labeling element as well as between a first labeling pad and a second labeling pad. The isolation layers are arranged according to different shared positions.

The "shared" means that a block element or a non-absorbent isolation layer needs to be arranged at the position where two liquid flow paths are split, i.e., at the area below the shared position, to allow the split liquid flow paths to flow independently, thereby achieving independent testing. For example, if a labeling area, or a labeling pad, or a labeling element is shared, flow splitting starts from the labeling area with a labeling substance, an isolation layer is arranged between a first testing element and a second testing element, and one end of the isolation layer contacts the labeling element. If a "liquid transfer element" is shared, an isolation layer is arranged starting from the liquid transfer element, such that liquid splitting starts from the liquid transfer element, and two liquid flow paths are split, preventing direct liquid exchange. Here, if both the liquid transfer element and the labeling element are "shared" (as shown in Fig. 15), flow splitting still starts from the labeling pad. If the labeling element is not shared, liquid splitting starts from the liquid transfer element, and isolation layers are arranged between upper and lower labeling elements and between testing elements, limiting communication of the liquid between the two fluid flow paths and interflowing.

In some embodiments, a test strip, as shown in Fig. 8 and Fig. 5, is arranged in a housing. A sample application hole 1005 and two testing windows are formed in the housing. The two testing windows are a first window 1007 for exposing testing areas on a first testing pad, and a second window 1006 for exposing testing areas on a second testing pad 802. Other areas are covered by the housing. The sample application hole 1005 corresponds to part of a flow splitting pad 90. In such a test device, as shown in Fig. 11, a liquid sample, e.g., a nasal swab liquid, is applied to the sample application hole 1005; after the liquid contacts the flow splitting pad 90, one liquid flow path flows on a first labeling pad and a first testing pad on a first test strip, and the other liquid flow path flows on, e.g., a second labeling area and a second testing pad, of a second test strip. Since both the testing pads expose the testing areas, the results of the testing areas can be read via the windows.

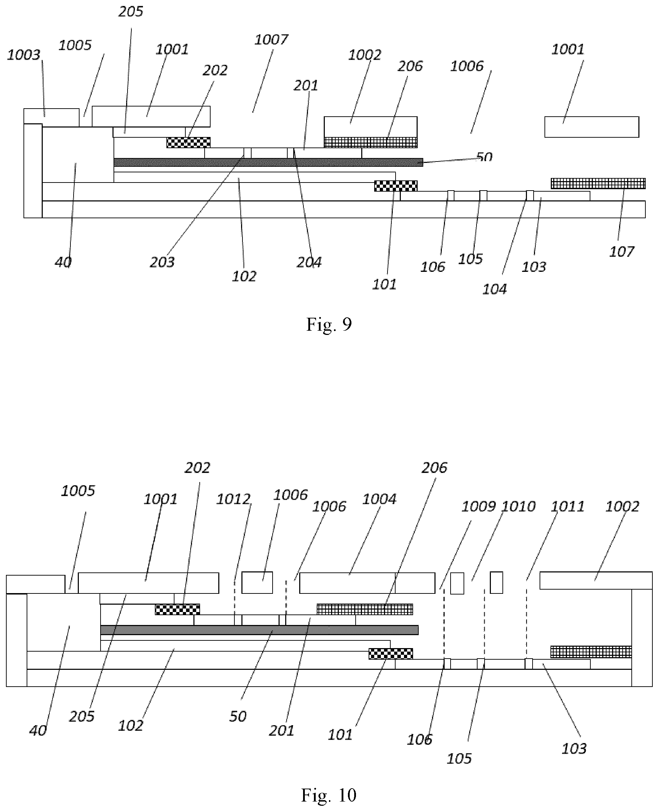

In some embodiments, a test strip, as shown in Fig. 9 and Fig. 4, is arranged in a housing. A sample application hole 1005 and two testing windows 1007 and 1006 are formed in the housing. The two testing windows are a first window 1007 for exposing testing areas 203 and 204 on a first testing element 201, and a second window 1006 for exposing testing areas 106 and 105 on a second testing element 103. Other areas are covered by the housing. The sample application hole 1005 corresponds to part of a flow splitting element 40. Such upper and lower test strips adhere together and are arranged in the housing. The sample application hole 1005 formed in the housing corresponds to the liquid flow splitting element 40 on the test strip. The other areas are arranged on the housing, e.g., an area 1001 between the sample application hole 1005 and the first window 1007, an area 1004 between the first window 1007 and the second window 1006, and an area between the second window 1006 and a housing tail 1002, are all closed; only the sample application hole, the first window 1007 and the second window 1006 are exposed, and the other parts are all closed. Viewed from the whole, it seems that only one conventional test strip is arranged in the housing, but actually, the liquid is split inside the test strip of the present disclosure. In this specific embodiment, a liquid sample, e.g., a nasal swab fluid, is applied to the sample application hole 1005; after the liquid contacts the flow splitting element 40 (which also functions as a liquid receiving element), one liquid flow path flows through a first liquid transfer element 205, a first labeling element 202, and testing areas 203 and 204 on a first testing element 201 on a first test strip, and flows on a first absorbent element 206 (if present), and the other liquid flow path flows through a second liquid transfer element 102, a second labeling element 101, and testing areas 106 and 105 on a second testing element 103. If a second absorbent element 107 is arranged, the liquid is also absorbed by the second absorbent element. If analytes are included in the sample, the color appears or does not appear on testing areas corresponding to two windows for representing a positive or negative result. For example, when an antigen is tested by double antibody sandwich assay, a labeling element is treated with a first antibody that specifically binds to the antigen and is conjugated with a colored particle, and a second antibody that specifically binds to the antigen is immobilized on testing areas. If the liquid sample includes a specific antigen to the analytes, lines appear on the testing areas; otherwise, no line appears. In one embodiment, the second testing component 103 includes a testing result control area 104 for testing the validity of the testing results of the test strip. Only one testing result control area is required to indicate the validity of the testing results on the two testing elements (as shown in the top view of Fig. 11, which is a schematic view of positive results for Influenza A and B, novel coronavirus, and RSV).

In some embodiments, a test strip, as shown in Fig. 10 and Fig. 4, is arranged in a housing. A sample application hole 1005 and five testing window areas are formed in the housing. The five testing window areas are two small windows for exposing two testing areas 203 and 204 on a first testing element 201, and three small windows for exposing testing areas 106 and 105 and a testing result control area on a second testing element 103. Thus, each testing area is provided with a window, and other parts are all covered to be not exposed. For example, a window 1012 is formed in the testing area 203, a window 1006 is formed in the testing area 204, and the part between the two windows is also covered by the element on the housing. A window 1009 is formed in the testing area 106 on the second testing element 103, a window 1010 is formed in the testing area 105, a window 1011 is formed in the testing result control area, and other parts are all covered by the housing. The sample application hole 1005 corresponds to part of the liquid flow splitting element 40. Such upper and lower test strips adhere together and are arranged in the housing. The sample application hole 1005 formed in the housing corresponds to the liquid flow splitting element 40 on the test strip. Viewed from the whole (Fig. 13), it seems that only one conventional test strip is arranged in the housing, but actually, the liquid is split inside the test strip of the present disclosure (Fig. 13). In this specific embodiment, a liquid sample, e.g., a nasal swab fluid, is applied to the sample application hole 1005; after the liquid contacts the flow splitting element 40 (which also functions as a liquid receiving element), one liquid flow path flows through a first liquid transfer element 205, a first labeling element 202, and testing areas 203 and 204 on a first testing element 201 on a first test strip; testing results are displayed on the corresponding testing areas on the windows 1012 and 1006; the liquid flows ona first absorbent element 206 (if present); the other liquid flow path flows through a second liquid transfer element 102, a second labeling element 101, and testing areas 106 and 105 on a second testing element 103, such that testing results can be displayed on windows 1009, 1010, and 1011. If a second absorbent element 107 is arranged, the liquid is also absorbed by the second absorbent element. If analytes are included in the sample, the color appears or does not appear on testing areas corresponding to four windows for representing a positive or negative result. For example, when an antigen is tested by double antibody sandwich assay, a labeling element is treated with a first antibody that specifically binds to the antigen and is conjugated with a colored particle, and a second antibody that specifically binds to the antigen is immobilized on testing areas. If the liquid sample includes a specific antigen to the analytes, lines appear on the testing areas; otherwise, no line appears. In one embodiment, the second testing component 103 includes a testing result control area 104 for testing the validity of the testing results of the test strip. Only one testing result control area is required to indicate the validity of the testing results on the two testing elements (as shown in Fig. 15, which shows positive results for testing Influenza A and B, novel coronavirus, and RSV, with colored lines in each window).

The following embodiments of the present disclosure also belong to a part of the present disclosure.

Provided is a test device, including a first liquid flow path and a second liquid flow path; one or more testing areas are arranged on the first liquid flow path; and one or more testing areas are also arranged on the second liquid flow path, wherein the first liquid flow path is located above the second liquid flow path, and the first and second liquid flow paths are not in liquid communication.

In some embodiments, the first liquid flow path includes a first testing element; the second liquid flow path includes a second testing element; and the testing areas are arranged on the first or second testing element.

In some embodiments, the first liquid flow path and the second liquid flow path share a reagent area, and a non-absorbent isolation layer is arranged between the first liquid flow path and the second liquid flow path. In some embodiments, the isolation layer isolates the first liquid flow path from the second liquid flow path, starting from the shared reagent area. In some embodiments, the shared reagent area includes a labeling element, or a liquid transfer element upstream of the labeling element.

In some embodiments, the first liquid flow path includes a first labeling element and a first testing element; and the second liquid flow path includes a second labeling element and a second testing element. In some embodiments, the first liquid flow path and the second liquid flow path share a liquid transfer element, and a liquid is split on the liquid transfer element. In some embodiments, the isolation layer starts flow splitting from the liquid transfer element and isolates the first liquid flow path from the second liquid flow path, preventing mutual flowing. In some embodiments, the first liquid transfer element is in contact with the second liquid transfer element, thereby achieving liquid communication. In some embodiments, the contact portion is used for receiving the liquid sample.

In some embodiments, the first liquid flow path includes a first labeling element, a first testing element, and a first liquid transfer element located upstream of the first labeling element; and the second liquid flow path includes a second labeling element, a second testing element, and a second liquid transfer element located upstream of the second labeling element. In some embodiments, the first liquid flow path and the second liquid flow path share a liquid flow splitting element, and a liquid is split on the liquid flow splitting element. In some embodiments, the isolation layer starts flow splitting from the liquid flow splitting element and isolates the liquid sample between the first liquid flow path and the second liquid flow path, preventing mutual flowing. The flow splitting element is arranged to receive the liquid sample and be in fluid communication with the first liquid transfer element and the second liquid transfer element.

In some embodiments, the first liquid flow path covers the second liquid flow path, or the material through which the first liquid flow path flows covers the material through which the second liquid flow path flows. In some embodiments, the material is a porous absorbent material and has capillary force. In some embodiments, testing areas on the first liquid flow path do not cover testing areas on the second liquid flow path, and the testing areas on both the liquid flow paths are exposed and uncovered. In some embodiments, the material through which the first liquid flow path flows includes a first lateral flow test element, and the material through which the second liquid flow path flows includes a second lateral flow test element. In some embodiments, the first lateral flow test element does not cover the area where testing areas are arranged on the testing pad of the second lateral flow test element. In some embodiments, the first lateral flow test element covers the second lateral flow test element, wherein the length of the first lateral flow test element is substantially equal to the sum of the lengths of the second liquid transfer element and the labeling pad on the second lateral flow test element.

In some embodiments, the present disclosure provides a device for testing an analyte in a liquid sample. The device includes a first lateral flow test element including a first liquid transfer element, a first labeling element, and a first testing element; and a second lateral flow test element including a second liquid transfer element, a second labeling element, and a second testing element, wherein the first liquid transfer element is in liquid communication with the second liquid transfer element, a first labeling element is not in liquid communication with a second labeling element, and a second testing element is not in liquid communication with a first testing element. In some embodiments, the second testing element is located below the first testing element, and the second testing element and the first testing element are connected into an integrated structure. In some embodiments, the first liquid transfer element and the second liquid transfer element are arranged for simultaneously receiving the liquid sample. In some embodiments, the device further includes a sample pad for receiving the liquid sample, and the sample pad is in fluid communication with the first liquid transfer element and the second liquid transfer element. In some embodiments, a non-absorbent isolation layer is arranged between the first lateral flow test element and the second lateral flow test element. In some embodiments, a non-absorbent isolation layer is arranged between the first labeling pad and the second labeling pad. In some embodiments, a non-absorbent isolation layer is arranged between the first testing pad and the second testing pad. In some embodiments, a non-absorbent isolation layer is arranged between the first labeling pad and the second labeling pad, and a non-absorbent isolation layer is arranged between the first testing pad and the second testing pad.

The present disclosure provides a method for testing an analyte in a liquid sample. The method includes: providing a test device, wherein the device includes: a first lateral flow test element, including a first liquid transfer element, a first labeling element, and a first testing element; and a second lateral flow test element, including a second liquid transfer element, a second labeling element, and a second testing element; allowing the first liquid transfer element and the second liquid transfer element to simultaneously receive the liquid sample; allowing part of a liquid to flow along the first lateral flow test element; and allowing the other part of the liquid to flow along the second lateral flow test element, wherein the liquid flowing on the first lateral flow test element does not come into exchange with the liquid flowing on the second lateral flow test element.

The present disclosure provides a method for testing an analyte in a liquid sample. The method includes: providing a test device, wherein the device includes: a first lateral flow test element, including a first liquid transfer element, a first labeling element, and a first testing element; and a second lateral flow test element, including a second liquid transfer element, a second labeling element, and a second testing element; and allowing the first liquid transfer element and the second liquid transfer element to simultaneously receive the liquid sample, wherein liquids flow independently on the first lateral flow test element and the second lateral flow test element.

The present disclosure provides a method for testing an analyte in a liquid sample. The method includes: providing a test device, wherein the device includes: a first lateral flow test element, including a first liquid transfer element, a first labeling element, and a first testing element; and a second lateral flow test element, including a second liquid transfer element, a second labeling element, and a second testing element; allowing the first liquid transfer element and the second liquid transfer element to simultaneously receive the liquid sample; and allowing a liquid flowing through the first labeling element and the first testing element to be not substantially the same as a liquid flowing through the second testing element and the second labeling element.

In some embodiments, the first lateral flow test element is allowed to be located above the second lateral flow test element. In some embodiments, an isolation layer is arranged between the first lateral flow test element and the second lateral flow test element. In some embodiments, the first lateral flow test element and the second lateral flow test element are connected or adhere together via the isolation layer. In some embodiments, a non-absorbent isolation layer is arranged between the first labeling pad and the second labeling pad. In some embodiments, a length of the first lateral flow test element is allowed to be smaller than a length of the second lateral flow test element. In some embodiments, a length of the first lateral flow test element is allowed to be equal to or smaller than a sum of lengths of the second liquid transfer element and the second labeling element on the second lateral flow test element. In some embodiments, the isolation layer is arranged between the first lateral flow test element and the second liquid transfer element and the second labeling element on the second lateral flow test element. In some embodiments, the first lateral flow test element and the second lateral flow test element adhere into an integrated structure via the isolation layer.

All patents and publications mentioned in the specification of the present disclosure indicate that these are disclosed techniques in the art and can be used by the present disclosure. All patents and publications cited herein are likewise listed in the references as if each publication is specifically and separately referenced. The present disclosure described herein may be implemented in the absence of any one or more elements, and one or more limitations, which are not specifically stated herein. For example, the terms "comprising", "essentially consisting of', and "consisting of' in each example here may be replaced by the rest 2 terms. The term "a/an" here merely means "one", but does not exclude including 2 or more instead of including only one. The terms and expressions employed here are descriptive and are not limited thereto, and there is no intention here to indicate that the terms and interpretations described herein exclude any equivalent features, but it can be noted that any appropriate changes or modifications can be made within the scope of the present disclosure and claims. It can be understood that the embodiments described in the present disclosure are preferred embodiments and features, and any person skilled in the art can make some modifications and changes based on the essence of the description of the present disclosure. These modifications and changes are also considered to be within the scope of the present disclosure and the scope limited by the independent claims and the dependent claims.

## Claims

1. A device for testing multiple analytes in a liquid sample, comprising:
a first testing element comprising one or more testing areas thereon;
a second testing element comprising one or more testing areas thereon; and
a liquid distribution element, wherein the liquid distribution element keeps fluid communication with the first testing element and the second test element; and wherein the first testing element is located above the second test element.

2. The device according to claim 1, wherein the first testing element covers the second testing element thereon.

3. The device according to any one of claims 1-2, wherein the first testing element covers upstream of the second testing element.

4. The device according to any one of claims 1-3, wherein the first testing element is not in liquid communication with the second testing element.

5. The device according to any one of claims 1-4, wherein a non-absorbent layer is arranged between the first testing element and the second testing element, such that a liquid on the first testing element does not flow on the second testing element.

6. The device according to any one of claims 1-5, wherein the liquid distribution element comprises a labeling substance; the labeling substance comprises a plurality of first antibodies that specifically bind to the multiple analytes separately; each first antibody is conjugated with a color-labeled particle; and a plurality of second antibodies are immobilized in the testing areas of the first testing element and the second testing element.

7. The device according to any one of claims 1-6, wherein a first labeling element is arranged upstream of the first testing element; a second labeling element is arranged upstream of the second testing element; and the first testing element and the first labeling element cover the second labeling pad but do not cover the testing area of the second testing element.

8. The device according to any one of claims 1-7, wherein a first labeling element is arranged upstream of the first testing element; a second labeling element is arranged upstream of the second testing element; both of the first testing element and the first labeling element have a projection area on the second testing element and the second labeling element; wherein the second labeling element is located within the projection area; and the testing area on the second testing element is not located within the projection area.

9. The device according to claim 8, wherein the non-absorbent layer is arranged within the projection area.

10. The device according to any one of claims 5-9, wherein the non-absorbent layer has an upper surface and a back surface; each surface has an adhesive layer; the adhesive layer of the upper surface of the layer adheres to the first testing element and the second labeling element; and the adhesive layer of the back surface of the layer adheres to the projection area.

11. The device according to any one of claims 7-10, wherein the first labeling element and the first testing element are located on a first support plate; the second labeling element and the second testing element are located on a second support plate; the first support plate has a projection area on the second support plate; and the testing area on the second testing element is not located within the projection area.

12. The device according to claim 11, wherein a physical spatial distance exists between the first support plate and the second support plate, such that a liquid flowing on the first labeling element and the first testing element is not capable of flowing onto the second labeling element and the second testing element.

13. The device according to any one of claim 7-12, wherein a first liquid transfer element is further included upstream of the first labeling element; a second liquid transfer element is further included upstream of the second labeling element; and the non-absorbent layer is arranged between the first liquid transfer element and the second liquid transfer element.

14. The device according to claim 14, wherein the first liquid transfer element and the second liquid transfer element keep liquid communication separately with the liquid distribution element.

15. The device according to any one of claims 1-14, when a liquid sample is connected with the liquid distribution element, a first liquid flowing on the first test element, a second liquid flowing on the second test element, and wherein the first liquid and the second liquid flow on the first teat element and second test element separately and independently.
